(19) Europäisches Patentamt
European Patent Office
Office européen des brevets

(11) EP 4 197 993 A1

(12) **EUROPEAN PATENT APPLICATION**

(43) Date of publication:
**21.06.2023 Bulletin 2023/25**

(21) Application number: **21214263.2**

(22) Date of filing: **14.12.2021**

(51) International Patent Classification (IPC):
***C07C 29/151** (2006.01)* ***C07C 31/04** (2006.01)*
***B01J 19/00** (2006.01)*

(52) Cooperative Patent Classification (CPC):
(C-Sets available)
**B01J 19/00; C07C 29/1512** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BASF SE**
**67056 Ludwigshafen am Rhein (DE)**

(72) Inventors:
- **Britzius, Susanne**
  **67056 Ludwigshafen (DE)**
- **Vicari, Maximilian**
  **67056 Ludwigshafen (DE)**

(74) Representative: **BASF IP Association**
**BASF SE**
**GBI-C006**
**67056 Ludwigshafen (DE)**

(54) **INTEGRATED PLANT AND PROCESS FOR THE PRODUCTION OF METHANOL FROM CARBON DIOXIDE AND HYDROGEN**

(57) An integrated plant and a process for the production of methanol from a carbon dioxide and hydrogen, and its start-up, in which an electrical heater or a heat exchanger connected to an electrical heater is used for the provision of thermal energy additionally required during the operation and start-up.

EP 4 197 993 A1

(52) Cooperative Patent Classification (CPC): (Cont.)

C-Sets
**C07C 29/1512, C07C 31/04**

## Description

The present invention relates to an integrated plant and a process for the production of methanol from a carbon dioxide and hydrogen, and its start-up, in which an electrical heater or a heat exchanger connected to an electrical heater is used for the provision of thermal energy additionally required during the operation and start-up.

Methanol is one of the most important synthesis raw materials globally. Beside its use as an important solvent, it is used for the syntheses of formaldehyde, acetic acid, methyl tert-butyl ether (MTBE), dimethyl terephthalate, methyl methacrylate, methylamines and various other compounds in large volumes.

Methanol is classically produced on the industrial scale from synthesis gas in a reactor in the presence of a methanol synthesis catalyst. The synthesis gas comprises mainly hydrogen and carbon monoxide, and, depending on the amount of production and workup, also corresponding amounts of carbon dioxide, water and what are called inert gases, for instance methane, nitrogen or argon.

According to Ullmann's Encyclopedia of Industrial Chemistry, "Methanol", 2012 Wiley-VCH Verlag GmbH & Co. KGaA, Weinheim, Germany, synthesis gas is converted to methanol typically in what is called the low-pressure process within a pressure range of 5 to 10 MPa over copper- and zinc-comprising methanol synthesis catalysts. This involves converting both carbon monoxide and carbon dioxide to methanol.

$$CO + 2H_2 \rightleftharpoons CH_3OH \qquad \Delta H_{300K} = -90.77 \text{ kJ/mol} \qquad (1)$$

$$CO_2 + 3H_2 \rightleftharpoons CH_3OH + H_2O \qquad \Delta H_{300K} = -49.16 \text{ kJ/mol} \qquad (2)$$

In addition to the two methanol-forming reactions (1) and (2), the endothermic reverse water-gas shift reaction of carbon dioxide and hydrogen has also to be taken into account.

$$CO_2 + H_2 \rightleftharpoons CO + H_2O \qquad \Delta H_{300K} = +41.21 \text{ kJ/mol} \qquad (3)$$

As a side reaction, methanation of carbon monoxide and carbon dioxide occurs under these reaction conditions, as it is for example described in Römpp Lexikon Chemie, "Methanisierung", 2019, Georg Thieme Verlag KG, Stuttgart.

$$CO + 3H_2 \rightleftharpoons CH_4 + H_2O \qquad (4)$$

$$CO_2 + 4H_2 \rightleftharpoons CH_4 + 2H_2O \qquad (5)$$

Based on reaction equations (1) and (2), the stoichiometric number S is found as follows for the methanol synthesis:

$$S = \frac{n(H_2) - n(CO_2)}{n(CO) + n(CO_2)} \qquad (6)$$

where n in each case represents the respective molar amounts. A stoichiometric number S of 2 corresponds to the theoretical number. However, a surplus of hydrogen, which is expressed by a stoichiometric number S of significantly above 2, has a beneficial effect on the performance of the methanol synthesis catalyst and it also shifts the reaction equilibrium to methanol.

The synthesis gas which is normally used in the classical methanol synthesis is typically obtained from natural gas, other streams comprising hydrocarbons and in some cases also by coal gasification or wood gasification. Standard preparation processes are steam reforming, autothermal reforming, combinations thereof or partial oxidation. All these

processes require a valuable carbon source and energy for their conversion to synthesis gas. Such energy is typically generated by the burning of a fossil fuel like natural gas. Thus, already the classical production of synthesis gas generates a substantial amount of carbon dioxide as a byproduct of the generation of the required heat for the conversion of raw materials into synthesis gas.

Alternatively, synthesis gas can also be produced by gasification of biomass or carbon containing waste streams. However, these resources are, compared with the demand of a commercial methanol plant, typically insufficient in their amount.

In the classical methanol synthesis as described in Ullmann's Encyclopedia of Industrial Chemistry, "Methanol" chapter, section 5.2.2, 2012 Wiley-VCH Verlag GmbH & Co. KGaA, Weinheim, Germany, No. 10.1002/14356007.a16_465.pub3, for the Lurgi MegaMethanol synthesis, synthesis gas is converted under heterogeneous catalysis to methanol in a methanol synthesis reactor, and a methanol-enriched raw methanol stream is first condensed out of the reaction mixture obtained. The nonconverted gaseous components are recycled to the synthesis reactor. An adequate amount of this gas stream is purged out to keep the content of the inert gases low and to support the reaction by a high partial pressure of the educt gases hydrogen, carbon monoxide and carbon dioxide. The methanol-enriched liquid stream obtained by the abovementioned condensing-out is subsequently expanded for outgassing. The further methanol-enriched liquid stream that remains after the outgassing is then subjected to a multistage distillation for the actual methanol recovery and pure distillation. The heat for the operation of the multistage distillation can be fully provided by the exothermic methanol synthesis step via a circulating thermo fluid.

According to section 7 of the before-mentioned article in Ullmann's Encyclopedia of Industrial Chemistry, high purity methanol for laboratory and technical use is typically specified by a methanol content of 99.0 wt.-% or higher. The same may also apply for methanol for synthesis use. The preparation of such high purity methanol requires a multistage distillation with at least two distillation columns. For the preparation of methanol with a considerably lower purity, such as for fuel application for which a purity of 95 wt.-% or less suffices, a one stage distillation is generally appropriate.

Although the classical processes for the synthesis of methanol on the basis of synthesis gas seem to be more or less optimized regarding energy consumption and carbon dioxide emission, there are increasing thoughts in the state of the art on how to reduce the carbon dioxide foot print of the whole methanol production chain as a such.

In this context, WO 2017/103,679 proposes an integrated system for the production of methanol based on synthesis gas generated by autothermal reforming of methane, in which a part of the required thermal energy for the autothermal reforming is provided as electrical energy generated by renewable energy sources such as solar, wind, geothermal, hydroelectricity or tidal energy sources.

Although the mentioned process already reduces the formation of carbon dioxide by additionally using electrical energy from renewable sources in the autothermal reforming, it still requires methane as a fossil raw material, and it still converts in the autothermal reforming part of the methane to carbon dioxide.

A great advantage for a more environmental friendly production of methanol is the replacement of fossil based synthesis gas by carbon dioxide and hydrogen.

E. Van-Dal et al. in Chemical Engineering Transactions 29 (2012) 463-468 investigated the energy balance of a hypothetical methanol plant based on carbon dioxide from flue gas of a coal power plant, and hydrogen from electrolysis of water using $CO_2$-free electricity, comprising an adiabatic reactor and a one column distillation by a simulation with Aspen Plus™ software. The simulation showed that 46% of the steam necessary for the $CO_2$ capture can be provided by the methanol plant, and that no external heat was required. Considering the hydrogen production by electrolysis of water with $CO_2$-free electricity, such hypothetical plant avoids not only the direct use of fossil based raw material as the flue gas is only an unavoidable by-product of a power plant, but also significantly reduces the emission of carbon dioxide.

At the first glance, this concept may look advantageous as it enables the production of methanol from carbon dioxide and hydrogen without using additional fossil raw material and without using additional fossil-based thermal energy. However, at the second glance, it is to emphasize that the hypothetical methanol plant has at least one crucial disadvantage, namely the existence of only one distillation column. Such a one column distillation practically only enables the production of low purity methanol with an assumed purity of 95 wt.-% or less, which may be useful for fuel purposes but not for the important use as a synthesis component in the chemicals value chain. Consequently, the finding that no external heat was required, only refers to the investigated process with a one column distillation.

Another Aspen Plus™ simulation of a hypothetical methanol plant based on carbon dioxide and hydrogen is described by C. Chen et al. in Applied Energy 243 (2019) 71-90. Although they also selected a one column distillation, they simulated a methanol purity of 99.8 wt.-%. They realized that such a plant then required additional heat and proposed to add it by heat pumps. The authors see the advantage of heat pumps in requiring only a minimal modifications to current industrial processes, so that they can easily be retrofitted to conventional plants, and clearly dis-advise on page 72, right column, lines 8 to 11 from using electricity for heating as being inefficient. However, the authors are silent on how such concept using heat pumps should work in detail.

Although only a one column distillation was mentioned, C. Chen et al. specified the methanol purity as 99.8 wt.-%. This indicates that the simulated distillation column might have been a huge column with a high number of

theoretical stages requiring a very high reflux. Such huge columns and distillation conditions require significantly more energy for their operation than usual distillation columns. This is consistent with the finding of C. Chen et al., according to that the simulated methanol plant based on carbon dioxide and hydrogen, and producing high purity methanol with a purity of 99.8 wt.-% requires additional energy for the operation of the distillation step, whereas a methanol plant based on synthesis gas does not require additional energy for the production of high purity methanol. This is caused by the fact that the methanol formation from carbon dioxide is less exothermic than from carbon monoxide, as can be seen by the enthalpies mentioned in the above reaction equations (1) and (2). According to reaction equation (1), the enthalpy $\Delta H_{300K}$ of the hydrogenation of carbon monoxide is -90.77 kJ/mol, whereas $\Delta H_{300K}$ of the hydrogenation of carbon dioxide is only -49.16 kJ/mol as shown in reaction equation (2). Hence, less steam is produced in case of using carbon dioxide and hydrogen, which then does no more suffice to fully heat up the stream for the distillation to high purity methanol, and C. Chen et al. propose to use heat pumps to balance the difference.

However, heat pumps are complex in their design, requiring a compressor as a rotating equipment and thus an increased maintenance as well as an increased risk of malfunction. Moreover, and this might be a serious disadvantage in practice, heat pumps are sluggish in case load adaptions are required as it would be during start-up, but also during normal operation if the supply with carbon dioxide, hydrogen or electrical energy would not remain constant, as it might be the case for a wind or solar power operated plant.

It was an object of the present invention to find an integrated plant for the production of methanol from a carbon dioxide and hydrogen containing stream, and a process for its operation and its start-up, which enables the production of methanol in a purity of ≥ 98 wt.-% in a high yield, and for which the additional heat required for the operation of the purification section as well as the heat required for the star-up can be easily provided by an environmental friendly, renewable energy source without using fossil-based energy carriers. Furthermore, the integrated plant shall largely use the same apparatuses and interconnections of established methanol synthesis plants customary in the industry and which hitherto have been based on the use of synthesis gas and the addition of conventional, fossil-based energy, so that such plants can easily be modified. Moreover, the integrated plant shall be easy to operate, even under difficult conditions under which the environmental friendly energy source shows considerable fluctuations.

We have found an integrated plant for the production of methanol from a carbon dioxide and hydrogen containing stream, comprising

(a) a methanol synthesis unit (A) containing a methanol synthesis reactor unit (A1), capable of being cooled or heated by a thermo fluid and having a thermo fluid inlet and outlet, for the conversion of carbon dioxide and hydrogen into a methanol containing reaction mixture, and a separator unit (A2) for its separation into a methanol and water enriched raw methanol stream and a gaseous carbon dioxide, carbon monoxide and hydrogen containing stream, whereas the methanol synthesis unit (A) contains

- an inlet for the inlet of carbon dioxide and hydrogen,
- an outlet for the outlet of the methanol and water enriched raw methanol stream,
- an outlet for the outlet of the gaseous carbon dioxide, carbon monoxide and hydrogen containing stream,
- an inlet for the inlet of a thermo fluid, being interconnected with the thermo fluid inlet of the methanol synthesis reactor unit (A1), and
- an outlet for the outlet of the thermo fluid, being interconnected with the thermo fluid outlet of the methanol synthesis reactor unit (A1),

(b) a methanol recovery unit (B) containing a distillation unit for the separation of the methanol and water enriched raw methanol stream into a stream containing purified methanol, a stream containing components with a lower boiling point than methanol, and a stream containing components with a higher boiling point than methanol, whereas the distillation unit contains a methanol purification column and a heat exchanger for the provision of heat required for the operation of the methanol purification column and having a thermo fluid inlet and outlet, whereas the methanol recovery unit (B) contains

- an inlet for the provision with the methanol and water enriched raw methanol stream, whereas this inlet is interconnected with the outlet for the outlet of the methanol and water enriched raw methanol stream of the methanol synthesis unit (A),
- an outlet for the outlet of components having a lower boiling point than methanol,
- an outlet for the outlet of the purified methanol,
- an outlet for the outlet of components having a higher boiling point than methanol,
- an inlet for the inlet of a thermo fluid, being interconnected with the inlet of the heat exchanger, and
- an outlet for the outlet of the thermo fluid, being interconnected with the outlet of the heat exchanger,

(c) a thermo fluid system (C) containing a vessel (C1) for the storage and provision of the thermo fluid, whereas the thermo fluid system (C) contains

- an outlet for the outlet of the thermo fluid, whereas this outlet is interconnected with the inlet of the thermo fluid of the methanol synthesis unit (A),
- an inlet for the inlet of the thermo fluid, whereas this inlet is interconnected with the outlet of the thermo fluid of the methanol synthesis unit (A),
- an outlet for the outlet of the thermo fluid, whereas this outlet is interconnected with the inlet of the thermo fluid of the methanol recovery unit (B), and
- an inlet for the inlet of the thermo fluid, whereas this inlet is interconnected with the outlet of the thermo fluid of the methanol recovery unit (B),

whereas the thermo fluid system (C) together with the interconnections to and from the methanol synthesis unit (A) and the methanol recovery unit (B) and their flow paths therein provide a thermo fluid loop, and in which the thermo fluid system (C) further contains an electrical heater (EH) or a heat exchanger connected to an electrical heater (EH).

The integrated plant of the invention is based on plants for the continuous low-pressure synthesis of methanol from carbon dioxide and hydrogen known to the person skilled in the art, having

(a) a methanol synthesis unit, in which carbon dioxide and hydrogen are converted in a methanol synthesis reactor in the presence of a methanol synthesis catalyst to a reaction mixture from which a methanol and water enriched raw methanol stream is separated off,
(b) a methanol recovery unit, in which the methanol and water enriched raw methanol stream is further processed by distillation to obtain purified methanol, and
(c) a thermo fluid system, in which a thermo fluid is circulated to remove heat produced in the methanol synthesis reactor and to provide heat required in the methanol recovery unit.

As known from the state of the art, such plants require additional energy for the operation of the methanol recovery unit if purified methanol with a purity of ≥ 98 wt.-% is intended to be produced, since the heat produced in the methanol synthesis reactor is less than the heat required in the methanol recovery unit. The core of the invention is the provision of an electrical heater or a heat exchanger connected to an electrical heater as an integral part of the thermo fluid system. Such an electrical heater can be operated with environmental friendly electricity generated by renewable sources and thus without producing climate-damaging emissions. Furthermore, such an electrical heater can easily and flexibly be used for the provision of the additionally required heat during the normal operation of the integrated plant as well as for the provision of the heat required for the start-up.

The integrated plant of the invention is elucidated in detail hereinafter.

Fig. 1 shows the block diagram of a general embodiment of the integrated plant, in which the interconnection between the methanol synthesis unit (A), the methanol recovery unit (B) and the thermo fluid system (C) is illustrated. The labels therein have the following meanings:

(A) methanol synthesis unit
(B) methanol recovery unit
(C) thermo fluid system
(EH) electrical heater or heat exchanger connected to an electrical heater

(I) carbon dioxide and hydrogen containing stream
(II) methanol and water enriched raw methanol stream
(III) carbon dioxide, carbon monoxide and hydrogen containing stream
(IV) low boiler stream
(V) purified methanol stream
(VI) high boiler stream

The methanol synthesis unit (A) contains a methanol synthesis reactor unit (A1), capable of being cooled or heated by a thermo fluid and having a thermo fluid inlet and outlet, for the conversion of carbon dioxide and hydrogen into a methanol containing reaction mixture, and a separator unit (A2) for its separation into a methanol and water enriched raw methanol stream and a gaseous carbon dioxide, carbon monoxide and hydrogen containing stream, as well as

- an inlet for the inlet of carbon dioxide and hydrogen,

- an outlet for the outlet of the methanol and water enriched raw methanol stream,
- an outlet for the outlet of the gaseous carbon dioxide, carbon monoxide and hydrogen containing stream,
- an inlet for the inlet of a thermo fluid, being interconnected with the thermo fluid inlet of the methanol synthesis reactor unit (A1), and
- an outlet for the outlet of the thermo fluid, being interconnected with the thermo fluid outlet of the methanol synthesis reactor unit (A1).

The block diagram in Fig. 1 shows the methanol synthesis unit (A) as a part of the integrated plant.

In principle, the methanol synthesis reactor unit (A1) can contain one or more methanol synthesis reactors. Generally, any reactors which are suitable for the exothermic conversion of carbon dioxide and hydrogen to methanol, and which are capable of being cooled or heated by a thermo fluid can be used. More specifically, any reactors which can typically be used for the conventional methanol process based on synthesis gas can be used. Such reactors are well known to the person skilled. As a preferred example of such reactors, quasi-isothermal reactors as described in Ullmann's Encyclopedia of Industrial Chemistry, "Methanol" chapter, Section 5.2.1 "Reactor Design", 2012 Wiley-VCH Verlag GmbH & Co. KGaA, Weinheim, Germany, are mentioned. Specific examples of quasi-isothermal reactors are variobar reactors and, what are called, double-wall superconverters.

The methanol synthesis reactor unit (A1) can contain a single reactor as well as an interconnection of two or more reactors. If two or more reactors are used, they can be interconnected in parallel, in series, or be interlaced with each other such as described for the Lurgi MegaMethanol process in the above-mentioned article in Ullmann's Encyclopedia of Industrial Chemistry. Typically, the methanol synthesis unit (A) contains usually 1 to 6 reactors, preferably 1 to 4 reactors, and more preferably 1 to 2 reactors.

The methanol synthesis reactor unit (A1) contains an inlet and an outlet for the thermo fluid, so that the thermo fluid will be able to cool or heat the methanol synthesis reactor. Furthermore, it is self-explanatory that the methanol synthesis reactor unit (A1) also contains an inlet for the carbon monoxide and hydrogen containing stream as well as an outlet for the reaction mixture. In case of a single reactor, the inlets and outlets of the methanol synthesis reactor unit (A1) refer to the inlets and outlets of the single reactor. In case of an interconnection of two or more reactors, at least the respective interconnection of the methanol synthesis reactor unit (A1) contains an inlet and an outlet for the thermo fluid as well as an inlet for the carbon monoxide and hydrogen containing stream and an outlet for the reaction mixture, with an adequate interconnection within the methanol synthesis reactor unit.

The separator unit (A2) serves as a unit for separating the reaction mixture into a methanol and water enriched raw methanol stream and a gaseous carbon dioxide, carbon monoxide and hydrogen containing stream. This is usually performed by cooling the reaction mixture down to enable the condensation of the methanol and water enriched raw methanol stream, and by subsequently separating the obtained gas/liquid mixture into a gaseous stream and a liquid stream. Hence, the separator unit (A2) is a unit which enables such a separation. The separator unit (A2) can contain one or more separators.

As a simple separator, an apparatus is mentioned, in which the reaction mixture can be cooled down and separated into a gas phase and a liquid phase, and which therefore contains an inlet for the reaction mixture, an outlet for the separated gas phase and a further outlet for the separated liquid phase. The cooler can be integrated in the separator or be a separate apparatus interconnected with the separator and being placed in flow direction before the separator.

If the separator unit (A2) contains more than one separator, the separators are usually interconnection in series. In such an interconnection, the gaseous stream of the preceding separator is guided through a cooler and fed to the inlet of the subsequent separator to enable a successive separation. The liquid streams of the separators are collected together. Preferably, the methanol synthesis unit (A) contains 1 to 5 subsequent separators, more preferably 1 to 4 separators and particularly preferably 1 to 3 separators in the separator unit (A2).

As the separated gaseous stream contains the valuable educts carbon dioxide, carbon monoxide and hydrogen, the methanol synthesis unit (A) typically also contains an interconnection between the outlet for the separated gas phase of the separator unit (A2) and the inlet of the methanol synthesis reactor unit (A1) to be able to recycle this gas stream.

Coming back to the methanol synthesis unit (A), the unit contains

- an inlet for the inlet of carbon dioxide and hydrogen,
- an outlet for the outlet of the methanol and water enriched raw methanol stream,
- an outlet for the outlet of the gaseous carbon dioxide, carbon monoxide and hydrogen containing stream,
- an inlet for the inlet of a thermo fluid, being interconnected with the thermo fluid inlet of the methanol synthesis reactor unit, and
- an outlet for the outlet of the thermo fluid, being interconnected with the thermo fluid outlet of the methanol synthesis reactor unit.

It is self-explanatory that

- the inlet for the inlet of carbon dioxide and hydrogen to the methanol synthesis unit (A) is interconnected with the inlet of carbon dioxide and hydrogen to the methanol synthesis reactor unit,
- the outlet for the reaction mixture of the methanol synthesis reactor unit (A1) interconnected with the inlet for the reaction mixture of the separator unit (A2),
- the outlet for the gaseous carbon dioxide, carbon monoxide and hydrogen containing stream of the separator unit (A2) interconnected with the outlet for the outlet of the gaseous carbon dioxide, carbon monoxide and hydrogen containing stream of the methanol synthesis unit (A), and
- the outlet for the methanol and water enriched raw methanol stream of the separator unit (A2) interconnected with the outlet for the methanol and water enriched raw methanol stream of the methanol synthesis unit (A).

A preferred interconnection within the methanol synthesis unit (A) is shown in Fig. 2. Stream (I) enters the methanol synthesis unit (A) and therein the methanol synthesis reactor (A1). The reaction mixture leaves the methanol synthesis reactor unit (A1) as stream (IIIa) and enters the separator unit (A2). The methanol and water enriched raw methanol stream leaves the separator unit (A2) as stream (II). One part of the gaseous carbon dioxide, carbon monoxide and hydrogen containing stream leaves the separator unit (A2) as stream (III), and another part of the carbon dioxide, carbon monoxide and hydrogen containing stream is recycled back to the methanol synthesis reactor unit (A1) as stream (IIIb). Moreover, thermo fluid from the thermo fluid system (C) enters the methanol synthesis unit (A) and therein the methanol synthesis reactor unit (A1), and thermo fluid from the methanol synthesis reactor unit (A1) leaves the methanol synthesis unit (A) to the thermo fluid system (C).

The methanol recovery unit (B) contains a distillation unit for the separation of the methanol and water enriched raw methanol stream into a stream containing purified methanol, a stream containing components with a lower boiling point than methanol, and a stream containing components with a higher boiling point than methanol, whereas the distillation unit contains a methanol purification column and a heat exchanger for the provision of heat required for the operation of the methanol purification column and having a thermo fluid inlet and outlet, as well as

- an inlet for the provision with the methanol and water enriched raw methanol stream, whereas this inlet is interconnected with the outlet for the outlet of the methanol and water enriched raw methanol stream of the methanol synthesis unit (A),
- an outlet for the outlet of components having a lower boiling point than methanol,
- an outlet for the outlet of the purified methanol,
- an outlet for the outlet of components having a higher boiling point than methanol,
- an inlet for the inlet of a thermo fluid, being interconnected with the inlet of the heat exchanger, and
- an outlet for the outlet of the thermo fluid, being interconnected with the outlet of the heat exchanger.

The block diagram in Fig. 1 shows the methanol recovery unit (B) as a part of the integrated plant.

The methanol purification column is a distillation column from which purified methanol is withdrawn.

Depending on the configuration of the methanol recovery unit (B), the distillation unit can contain one single distillation column for a one step separation of the methanol and water enriched raw methanol stream into a purified methanol stream, a stream containing components with a lower boiling point than methanol, and a stream containing components with a higher boiling point than methanol, or multiple interconnected distillation columns for a multiple step separation into the mentioned streams. Irrespective of whether a one-step or multi-step distillation is configured, the person skilled in the art knows how to design the respective distillation columns based on their separation tasks. As typical internals, structured packings, random packings or trays are mentioned as examples. Each distillation column is typically interconnected with a top condenser for the condensation of the head stream, and a reboiler for the heating of the bottoms. The inlet of the heat exchanger for the methanol purification column is interconnected with the inlet for the thermo fluid of the methanol recovery unit (B), and the outlet of the mentioned heat exchanger is interconnected with the outlet for the thermo fluid of the methanol recovery unit (B).

If the distillation unit contains only a single distillation column, which is then per definition the methanol purification column, it typically contains a high number of theoretical stages and is designed for a high reflux, in order to perform the separation task. In case of such a single step separation, the use of a divided wall column is an advantageous option. Here too, the person skilled in the art knows how to design such a single step separation. The energy for the operation of the distillation column is provided by a heat exchanger. It is typically placed as a reboiler for the heating the bottoms. The heat exchanger has a thermo fluid inlet and outlet, so that it can be operated with thermo fluid.

Since the methanol and water enriched raw methanol stream provided by the methanol synthesis unit (A) typically contains a significant amount of carbon dioxide, and typically has a higher pressure than required and useful for the distillation, it is preferred to expand the methanol and water enriched raw methanol stream before providing it to

the distillation unit. Thus, the methanol recovery unit (B) preferably additionally contains an expansion unit interconnected between the inlet for the provision with the methanol and water enriched raw methanol stream of the methanol recovery unit (B) and the inlet of the distillation unit. The expansion is typically effected in an apparatus in which the gas phase and the liquid phase can be efficiently separated from one another. Typically, the apparatus is a liquid separator. Suitable apparatuses for this purpose are known to those skilled in the art.

Since the expansion unit generates so called expansion gas, the methanol recovery unit (B) further contains in this preferred embodiment also an outlet for the outlet of the expansion gas.

Although a one-step distillation as mentioned above would in principle technically suffice to process the methanol and water enriched raw methanol stream to obtain purified methanol, a two-step distillation is preferred as it is more efficient. Therefore, the integrated plant of the invention preferably contains in the methanol recovery unit (B) a distillation unit which further contains a low boiler column for the separation of components having a lower boiling point than methanol, being located upstream to the methanol purification column, and a heat exchanger for the provision of heat required for the operation of the low boiler column. The bottom stream of the low boiler column contains the methanol and components with a higher boiling point than methanol.

The low boiler column is typically designed as a single distillation column, although it is of course also possible to use two or more distillation columns in parallel or in series. In a preferred embodiment of the integrated plant, the bottoms of the low boiler column are heated-up in a heat exchanger with is simultaneously used as a cooler in the separator unit (A2), so that the heat of the reaction mixture can be used for the operation of the low boiler column.

The methanol purification column can be designed as a single distillation column, also including a divided wall column, as well as a combination of two or more distillation columns. In case a combination of two or more distillation columns is used, they can be interconnected in parallel, in series or be interlaced with each.

Since the heat required for the operation of the methanol purification column is a considerable factor in the heat balance of the integrated plant, it is generally advantageous to take steps to reduce its heat demand. Therefore, it is preferred to materialize the methanol purification distillation as a pressure swing distillation. Hence, the integrated plant of the invention preferably contains in the methanol recovery unit (B) a distillation unit which further contains a second methanol purification column being interconnected with the other methanol purification column for their use as a pressure swing distillation.

In the pressure swing distillation, two specifically interconnected distillation columns are used. The typical interconnection of the two distillation columns is ideally explained by the flow of the streams. Their flow is roughly explained in the following. The stream to be purified enters the first distillation and is separated into a head stream, which already is purified methanol, and a bottom stream containing further methanol and components with a higher boiling point than methanol. The first distillation column is interconnected with a heat exchanger for the provision of the heat required for the pressure swing distillation. The bottom stream of the first distillation column enters the second distillation and is separated into a head stream, which is also purified methanol, and a bottom stream containing components with a higher boiling point than methanol. The bottoms of the second distillation column are heated-up in a heat exchanger using the heat of the head stream of the first distillation column, so that typically no further heat is required. The second distillation column is operated at a pressure lower than that of the first distillation column.

Pressure swing distillations are well known in the state of the art and the person skilled in the art knows how to design a pressure swing distillation for the integrated plant of the invention, and how to operate it.

As mentioned above, components with a higher boiling point than methanol are separated off in the methanol purification column. Since these components contain the water formed in the conversion of carbon dioxide and hydrogen, as well as organic by-products such as ethanol, propanols or butanols, it is advantageous to separate off these components separately as a water containing high boiler stream and a by-products containing so-called middle boiler stream. Consequently, a methanol recovery unit (B) is preferred, in which the outlet for the outlet of components having a lower boiling point than methanol is divided into an outlet for the outlet of a higher boiler stream containing water and an outlet for the outlet of a middle boiler stream containing organic by-products.

A preferred interconnection within the methanol recovery unit (B) is shown in Fig. 2. Stream (II) enters the methanol recovery unit (B) and therein the expansion unit (B1). The released expansion gas leaves the methanol recovery unit (B) as stream (IVa). The degassed raw methanol stream (IVb) enters the low boiler column (B2). The low boiler stream separated therein leaves the methanol recovery unit (B) as stream (IVc). The methanol containing bottom stream leaves the low boiler column (B2) as stream (IVd) and enters the methanol purification column (B3), which preferably is materialized as a pressure swing distillation containing two distillation columns. From the methanol purification column (B3) three streams are withdrawn, which leave the methanol recovery unit (B) as a purified methanol stream (V), a middle boiler stream (VII) and a high boiler stream (VI). Moreover, thermo fluid from the thermo fluid system (C) enters the methanol recovery unit (B) and therein the heat exchanger of the methanol purification column (B3), and thermo fluid from the heat exchanger of the methanol purification column (B3) leaves the methanol recovery unit (B) to the thermo fluid system (C).

According to the invention, it was found that it is particularly advantageous to provide, or at least partly provide,

the additionally required heat for the operation of the integrated plant as well as for its start-up by an electrical heater or a heat exchanger connected to an electrical heater as a part of the thermo fluid system of the integrated plant.

In more detail, the thermo fluid system (C) contains a vessel (C1) for the storage and provision of the thermo fluid, as well as

- an outlet for the outlet of the thermo fluid, whereas this outlet is interconnected with the inlet of the thermo fluid of the methanol synthesis unit (A),
- an inlet for the inlet of the thermo fluid, whereas this inlet is interconnected with the outlet of the thermo fluid of the methanol synthesis unit (A),
- an outlet for the outlet of the thermo fluid, whereas this outlet is interconnected with the inlet of the thermo fluid of the methanol recovery unit (B), and
- an inlet for the inlet of the thermo fluid, whereas this inlet is interconnected with the outlet of the thermo fluid of the methanol recovery unit (B),

whereas the thermo fluid system (C) together with the interconnections to and from the methanol synthesis unit (A) and the methanol recovery unit (B) and their flow paths therein provide a thermo fluid loop, and whereas the thermo fluid system (C) further contains an electrical heater (EH) or a heat exchanger connected to an electrical heater (EH).

The block diagram in Fig. 1 shows the thermo fluid system (C) as a part of the integrated plant.

The thermo fluid system (C) is interconnected with the methanol synthesis unit (A) and the methanol recovery unit (B) and enables the flow of thermo fluid to these two units and back. At units where heat is produced, the thermo fluid can absorb it and transport it to the thermo fluid system (C), and at units where heat is required, the thermo fluid can transport the heat to the respective unit and give it off. If more heat is required in the integrated plant than produced, the electrical heater or the heat exchanger connected to an electrical heater can easily provide this additional heat.

Since the operation of the integrated plant as well as their start-up requires some reservoir of the thermo fluid as a kind of thermo buffer, the thermo fluid system (C) contains a vessel (C1) for the storage and provision of the thermo fluid. Such vessels can in principle be any containers such as drums which are able to store the thermo fluid under the pressure and temperature of operation. Typically, the thermo fluid is a water based fluid and the typical pressures and temperatures are in the range of 0.15 to 5 MPa abs and 198 to 264°C. Depending on the temperature and pressure, the water based thermo fluid exists as liquid, steam or a mixture thereof. For example, the water based thermo fluid in the vessel (C1) is during operation a mixture of liquid water and steam. Based on the function of the inlets and outlets, i.e. whether a liquid, gaseous or mixed stream is to be supplied or withdrawn, the person skilled in the art knows where to locate them in vessel (C1).

Generally, electrical heaters are apparatuses in which the thermo fluid flows through and is heated by passing electrically heated surfaces therein. Such surfaces can for example be heating coils or heating spirals with an electrical resistance heating inside. Based on the overall requirements, such as the nature of the thermo fluid, the pressure and temperature range and the thermal energy to be provided, the person skilled in the art knows how to design such an electrical heater.

Alternatively, also a heat exchanger connected to an electrical heater can be placed within the thermo fluid system (C) for the heating of the thermo fluid. The connection between the heat exchanger and the electrical heater can be performed by a circulating heating fluid which is heated by an electrical heater, e.g. an electrical heater as described above, and which gives off its heat in the heat exchanger. Although such a heating construction is alternatively possible, the direct heating of the thermo fluid within the thermo fluid system (C) by an electrical heater as described above, is preferred.

The electrical heater can in principle be integrated into the vessel (C1) or be a separate apparatus within the thermo fluid system (C) interconnected with the vessel (C1). Moreover, the thermo fluid system (C) can contain a single electrical heater or two or more electrical heaters. In case of two or more electrical heaters, they can be interconnected in parallel or in series, or they also can be placed at different locations within the interconnection of the thermo fluid streams inside thermo fluid system (C). Although the concept of having more than one electrical heater, such as one within the vessel (C1) and another outside the vessel (C1), is an alternative, a concept of having the electrical heater only outside the vessel (C1) is preferred. Preferably, the integrated plant only contains a single electrical heater outside the vessel (C1), but for specific reasons, such as if two or more smaller electrical heaters would be more efficient than one big electrical heater, it may be advantageous to use a two or more electrical heaters in parallel or series.

A preferred interconnection within the thermo fluid system (C) is shown in Fig. 2. In this preferred interconnection within the thermo fluid system (C),

- the outlet of the thermo fluid being interconnected with the inlet of the thermo fluid of the methanol recovery unit (B) is interconnected with an outlet of the vessel (C1),
- the inlet of the thermo fluid being interconnected with the outlet of the thermo fluid of the methanol recovery unit (B)

is interconnected with an inlet of the vessel (C1),

- the outlet of the thermo fluid being interconnected with the inlet of the thermo fluid of the methanol synthesis unit (A) is interconnected via a valve V1 with an outlet of the vessel (C1),
- the inlet of the thermo fluid being interconnected with the outlet of the thermo fluid of the methanol synthesis unit (A) is interconnected with an inlet of the vessel (C1),
- the electrical heater has an inlet and an outlet of the thermo fluid,
- the inlet for the inlet of the thermo fluid of the electrical heater (EH) or the heat exchanger connected to an electrical heater (EH) is interconnected with an outlet of the vessel (C1),
- the outlet for the outlet of the thermo fluid of the electrical heater (EH) or the heat exchanger connected to an electrical heater (EH) is interconnected through a valve V2 with an inlet of the vessel (C1), and
- the outlet for the outlet of the thermo fluid of the electrical heater (EH) or the heat exchanger connected to an electrical heater (EH) is interconnected through a valve V3 with the outlet of the thermo fluid being interconnected with the inlet of the thermo fluid of the methanol synthesis unit (A).

A further preferred interconnection within the thermo fluid system (C) is also based on the interconnection shown in Fig. 2, but with the difference that the electrical heater (EH) is replaced by a heat exchanger connected to an electrical heater (EH).

Typically, vessel (C1) contains several outlets and several inlets, which is expressed by using the terms "an outlet" and "an inlet". However, it is principally also possible to use a vessel (C1) with only one outlet and one inlet, although it is not preferred. Similarly, the electrical heater (EH) or the heat exchanger connected to an electrical heater (EH) may contain one or several outlets and inlets, although the terms "the outlet" and "the inlet" are used.

The preferred, above-mentioned interconnection containing the valves V1 to V3 has the great advantage of being very flexible in their operation, as it enables

- a closed thermo fluid loop between the methanol synthesis unit (A) and the vessel (C1) via valve V1, which is important during normal operation for removing the heat generated in the methanol synthesis unit (A) and transporting it to vessel (C1),
- a closed thermo fluid loop between the electrical heater (EH) or the heat exchanger connected to an electrical heater (EH) and the vessel (C1) via valve V2, which is important during normal operation for providing additional heat to vessel (C1), which is additionally required in the methanol recovery unit (B), and
- a close thermo fluid loop between the electrical heater (EH) or the heat exchanger connected to an electrical heater (EH), via valve V3, the methanol synthesis unit (A) and vessel (C1), which is important during start-up for providing heat to the methanol synthesis unit (A).

Depending on the placing of the vessel (C1), the methanol synthesis reactor unit (A1) and the electrical heater (EH) or the heat exchanger connected to an electrical heater (EH) with regard to their vertical location, the flow of the thermo fluid between them require the support of pumps or may even be possible without pumping support. As a rotating equipment, pumps require additional electrical energy, need maintenance, and have the risk of break downs. Therefore, a solution without requiring pumps is to be preferred. Such a solution is possible by using the thermosyphon effect. The thermosyphon effect uses the effect that fluids at a higher temperature have a lower density than fluids at a lower temperature. Consequently, fluids having a lower temperature tend to flow downwards by gravity, and fluids having a higher temperature tend to flow upwards. This effect can also be used for the circulation of the thermo fluid between

- the methanol synthesis reactor unit (A1) and the vessel (C1),
- the electrical heater (EH) or the heat exchanger connected to an electrical heater (EH) and the vessel (C1), and
- the electrical heater (EH) or the heat exchanger connected to an electrical heater (EH), the methanol synthesis reactor unit (A1) and the vessel (C1).

Therefore, an integrated plant is preferred, in which in the thermo fluid system (C)

- the vessel (C1) is located at a height above the thermo fluid outlet of the methanol synthesis reactor within the methanol synthesis reactor unit (A1) from which the thermo fluid flows out, and
- the electrical heater (EH) or the heat exchanger connected to an electrical heater (EH) is located at a height below the thermo fluid inlet of the methanol synthesis reactor within the methanol synthesis reactor unit (A1) from which the thermo fluid flows in.

The person skilled in the art knows how to place the vessel (C1) and the electrical heater (EH) or the heat exchanger connected to an electrical heater (EH) regarding their vertical height with respect to the vertical height of the

inlet and outlet of the methanol synthesis reactor within the methanol synthesis reactor unit (A1) to ensure an appropriate flow of the thermo fluid, depending on the nature of the thermo fluid, its temperature and pressure, and its required flow.

Such an arrangement enables the use of the thermosyphon effect and thus an operation without requiring pumps for facilitating the flow of the thermo fluid between the electrical heater (EH) or the heat exchanger connected to an electrical heater (EH), the methanol synthesis reactor unit (A1) and the vessel (C1).

As already mentioned above, the thermo fluid used in the integrated plant is typically a water based fluid. This easily enables its use for cooling and heating since water can easily be vaporized under the absorption of vaporization energy, and it can easily be condensed under the release of condensation energy, so that not only thermal energy is absorbed or provided, but also energy from the vaporization or condensation. To also make use of the vaporization energy and condensation energy, the temperature and pressure of the thermo fluid is preferably adapted to the specific requirements in the methanol synthesis reactor unit (A1) and the methanol recovery unit (B), especially the temperature conditions and the amount of heat to be absorbed or provided. Based on these thoughts, the thermo fluid supplied to the methanol synthesis unit (A) has favorably a higher temperature than the thermo fluid supplied to the heat exchanger of the methanol purification column. Consequently, also the pressure of the thermo fluid supplied to the methanol synthesis unit (A) is higher than the pressure of the thermo fluid supplied to the heat exchanger of the methanol purification column. This difference can easily be overcome by depressurizing the thermo fluid between the vessel (C1) and the heat exchanger of the methanol purification column, and by pressurizing the backflow of the thermo fluid to vessel (C1).

In a preferred embodiment of the integrated plant, a further unit called steam and condensate system (C2) is interconnected as a further part of the thermo fluid system (C) in the interconnection of the thermo fluid between the outlet of vessel (C1) and the inlet of the methanol recovery unit (B), and the interconnection of the thermo fluid between the outlet of the methanol recovery unit (B) and the inlet of vessel (C1). A block diagram of such a preferred interconnection is shown in Fig. 3. The steam and condensate system (C2) can for example in the easiest way be materialized by a further container having inlets and outlets to which the thermo fluid of vessel (C1) is depressurized and from which the flowback to vessel (C1) is withdrawn an pressurized.

For practical reasons, it is further preferred to exchange a part of the thermo fluid circulating in the integrated plant to avoid an accumulation of salts and other impurities. Thus, the thermo fluid system (C) preferably also contains an inlet for the provision of fresh thermo fluid, which is usually called boiler feed water, an outlet for the condensed thermo fluid, which has been in circulation, which is usually called blow down, and an outlet of gaseous thermo fluid, which is usually called flash steam. Furthermore, a part of the steam is required for the degassing of the boiler feed water. Hence, the thermo fluid system (C) typically also contains further inlets and outlets for the streams mentioned above. Depending on the arrangements preferred, these inlets and outlets can be located at the vessel (C1), at the steam and condensate system (C2), or somewhere else. The person skilled in the art is familiar with such systems and knows how where to locate these additional inlets and outlets.

Furthermore, we have found a process for the production of methanol by

(a) converting a carbon dioxide and hydrogen containing stream (I) in a methanol synthesis reactor unit (A1) at a temperature of 150 to 300°C and a pressure of 3 to 10 MPa abs in the presence of a methanol synthesis catalyst to a reaction mixture containing methanol, water, carbon dioxide, carbon monoxide and hydrogen, condensing out of said reaction mixture in a gas/liquid separator (A2) a methanol and water enriched raw methanol stream (II) and separating off a gaseous stream (III) comprising carbon dioxide, carbon monoxide and hydrogen,

(b) separating the methanol and water enriched raw methanol stream (II) in a methanol recovery unit (B) by distillation into

- a stream (IV) containing components having a lower boiling point than methanol,
- a stream (V) containing purified methanol, and
- a stream (VI) containing components having a higher boiling point than methanol,

whereas heat generated in the methanol synthesis reactor unit (A1) is removed from it by a thermo fluid, circulating from and to a thermo fluid system (C), and heat required in the methanol recovery unit (B) is provided by a thermo fluid, circulating from and to the thermo fluid system (C), and

wherein 20 to 100% of the difference between the heat required in the methanol recovery unit (B) and the heat generated in the methanol synthesis reactor unit (A1) is supplied to the thermo fluid by an electrical heater (EH) or a heat exchanger connected to an electrical heater (EH).

The process of the invention is based on a continuously operated methanol synthesis by the low-pressure process known to the person skilled in the art, in which conventionally synthesis gas is converted at a pressure of 3 to

10 MPa abs in the presence of a methanol synthesis catalyst at a temperature of 150 to 300°C to a methanol containing reaction mixture and subsequently worked up stepwise for isolation of the methanol, in which the synthesis gas is exchanged by carbon dioxide and hydrogen as raw material. As already explained above, in case of using carbon dioxide and hydrogen as raw material, the heat generated in the methanol synthesis reactor by using carbon dioxide and hydrogen as raw material is less than the heat generated in the methanol synthesis reactor by using synthesis gas. This causes a deficit in the energy balance of the plant, particularly if purified methanol with a methanol purity of 98 to 100 wt.-% is intended to be produced, if the plant is operated at a load much lower than the load for which the plant was designed for, or if the plant is to be started-up. The core of the invention is to use an electrical heater or a heat exchanger connected to an electrical heater to supply the additionally required energy.

The process of the invention is preferably performed in an integrated plant whose block diagram is shown in Fig. 1 and which is already explained above.

More preferably, the process of the invention is performed in an integrated plant whose block diagram is shown in Fig. 2 and which is also already explained above. However, the meanings of the labels used therein are summarized together for a better overview.

(A1) methanol synthesis reactor unit
(A2) separator unit
(B1) expansion unit
(B2) low boiler column
(B3) methanol purification column
(C1) vessel
(EH) electrical heater or heat exchanger connected to an electrical heater

(I) carbon dioxide and hydrogen containing stream
(II) methanol and water enriched raw methanol stream
(III) carbon dioxide, carbon monoxide and hydrogen containing stream
(IIIa) reaction mixture
(IIIb) carbon dioxide, carbon monoxide and hydrogen containing recycle stream
(IVa) expansion gas
(IVb) degassed raw methanol stream
(IVc) low boiler stream from low boiler column
(IVd) bottom stream from low boiler column
(V) purified methanol stream
(VII) middle boiler stream
(VI) high boiler stream

Particularly preferably, the process of the invention is performed in an integrated plant whose block diagram is shown in Fig. 3 and which is also already explained above. However, the meaning of the additional label used therein is given below for a better overview.

(C2) steam and condensate system

The carbon dioxide supplied with stream (I) can in principle come from a wide variety of different sources. Examples include the supply of carbon dioxide from power plants based on fossil fuels, steal production, cement industry, chemical plants in which carbon dioxide is a by-product, from plastics such as not recyclable plastic waste, from renewable sources such as based on biomass, or ambient air from which carbon dioxide can be extracted. In view of using environmental friendly sources, renewable sources such as based on biomass, or ambient air from which carbon dioxide can be extracted are preferred.

The hydrogen supplied with stream (I) can in principle come from a wide variety of different sources. Examples include the supply of hydrogen from other production plants in which hydrogen is formed deliberately or as a by-product, for example from steam crackers or refineries, from the processing of synthesis gas, from the cracking of hydrocarbons, for example the pyrolysis of methane and/or higher hydrocarbons, or from the electrolysis of water. In view of using environmental friendly sources, the electrolysis of water is preferred, and particularly preferred if the electricity used therefore is environmental friendly generated, such as by wind or solar power.

The supplied carbon dioxide and hydrogen containing stream (I) has generally a carbon dioxide and hydrogen content of 95 to 100 vol.-%. Depending on the source, water, carbon monoxide and inert gases such as nitrogen, argon and hydrocarbons may be present as impurities. The amount of stream (I) supplied as well as the molar ratio between hydrogen and carbon dioxide is advantageously to be adapted such that the favored stoichiometric number S is obtained

at the inlet of the methanol synthesis reactor, considering possible recycle streams.

In order to enable the conversion of the carbon dioxide and hydrogen containing stream (I) at a pressure of 3 to 10 MPa abs, the carbon dioxide and hydrogen containing stream (I) is typically compressed to the desired pressure by means of a compressor and converted in a methanol synthesis reactor under the conditions specified.

In general, the stoichiometric number S ranges from 1 to 5 and preferably from 2 to 5. However, in order to support the methanol synthesis catalyst with regard to its activity, conversion and selectivity, the stoichiometric number S is more preferably ≥ 3, and particularly preferably ≥ 3.4.

Methanol synthesis reactors (A1) which in principle can be used for the process of the invention are already described above in the description of the integrated plant. It is emphasized that a single reactor as well as an interconnection of two or more reactors can be used.

Methanol synthesis catalysts that may be used in the process of the invention are virtually any catalysts which are also suitable for the conversion of synthesis gas to methanol under the process conditions mentioned. Corresponding methanol synthesis catalysts are common knowledge to those skilled in the art. Examples of these include the copper- and zinc-containing heterogeneous catalysts that are mentioned in Ullmann's Encyclopedia of Industrial Chemistry, "Methanol" chapter, Section 4.2 "Catalysts", 2012 Wiley-VCH Verlag GmbH & Co. KGaA, Weinheim, Germany. In general, these also comprise further elements, for example aluminum, rare earths or chromium.

The conversion is preferably effected at a temperature of ≥ 170°C and more preferably at ≥ 190°C, and preferably at ≤ 280°C and more preferably at ≤ 260°C. With regard to the pressure, the conversion is effected preferably at ≥ 3.5 MPa abs, more preferably at ≥ 6 MPa abs and preferably at ≤ 9 MPa abs.

Since the conversion is exotherm, the methanol synthesis reactor has to be cooled. It is therefore interconnected with the thermo fluid system (C).

The reaction mixture (Ilia) produced in the methanol synthesis reactor unit (A1) typically contains methanol, water, unconverted carbon dioxide and hydrogen, carbon monoxide formed by the reverse water-gas shift reaction and various by-products. Typical by-products are for example dimethyl ether, ethanol, methyl formate, higher alcohols such as propanols and butanols, and in case of a very active catalyst also methane.

Since the reaction mixture (Ilia) typically contains a considerable amount of valuable hydrogen, carbon dioxide and carbon monoxide, a part of these gases is preferably at first separated off from the methanol and water enriched raw methanol stream. In this preferred operation mode, the reaction mixture (IIIa) is cooled down and the condensed methanol and water enriched raw methanol stream separated off in a separator and withdrawn as stream (II). Preferably, a separator unit (A2) is used for this separation, in which the reaction mixture is successively cooled and separated into the methanol and water enriched raw methanol stream (II) and a carbon dioxide, carbon monoxide and hydrogen containing stream. More preferably, the separator unit (A2) contains 2 to 5 successive separation steps, and particularly preferably 2 to 4 successive separation steps. The major part of the carbon dioxide, carbon monoxide and hydrogen containing stream is recycled back as stream (IIIb) to the methanol synthesis reactor (A1). A smaller part of the stream is withdrawn and discharged as stream (III) in order to counteract an accumulation of inert gasses such as nitrogen. The methanol and water enriched raw methanol stream (II) is guided to the methanol recovery unit (B).

In the methanol recovery unit (B), the methanol and water enriched raw methanol stream (II) is further processed and separated into

- a stream (IV) containing components having a lower boiling point than methanol,
- a stream (V) containing purified methanol, and
- a stream (VI) containing components having a higher boiling point than methanol.

The respective block diagram is shown in Fig. 1.

Although, the separation process can be performed as a single step in a single distillation column, a multi-step distillation using at least two distillation columns is preferred. Furthermore, since the distillative workup is typically performed at a lower pressure than that present in the methanol synthesis reactor (A1), the methanol and water enriched raw methanol stream (II) is preferably expanded in an expansion unit (B1) to a pressure of 0.1 to 2 MPa abs before it is further guided to the distillation. Such a preferred multi-step processing is shown in Fig. 2.

The expansion in the expansion unit (B1) is preferably effected at a pressure of ≥ 0.2 MPa abs and more preferably at ≥ 0.4 MPa abs, and preferably at ≤ 1.5 MPa abs and more preferably at ≤ 1 MPa abs. In general, the temperature of the expanded mixture is 0 to 150°C, preferably ≥ 10°C and more preferably ≥ 20°C, and preferably ≤ 120°C and more preferably ≤ 60°C.

By the expansion, expansion gas is released and discharged as stream (IVa). The expansion gas (IVa) comprises carbon dioxide, hydrogen, methanol, inert compounds such as nitrogen and low boiling by-products such as methyl formate.

The degassed raw methanol stream (IVb) is thus further enriched in methanol and water, but also comprises further components in accordance with the solubilities and vapor pressures of the components present in stream (II),

for example gases dissolved therein such as carbon dioxide and by-products such as methyl formate, ethanol and others. The degassed raw methanol stream (IVb) is guided to the distillation unit which preferably contains a low boiler column (B2) and a methanol purification column (B3).

In the low boiler column (B2), the degassed raw methanol stream (IVb) is separated into a low boiler stream (IVc), which mainly contains carbon dioxide but also methyl formate, water and dimethyl ether, and a bottom stream (IVd), which mainly contains methanol and water, but also middle and high boiling by-products. The person skilled in the art knows how to operate such a low boiler column. Typically, the low boiler column (B2) is operated at a pressure of 0.1 to 0.5 MPa abs. The low boiler stream (IVc) is discarded, and the bottom stream (IVd) guided to the methanol purification column (B3).

The energy for the operation of the low boiler column (B2) is provided by a heat exchanger, which is advantageously heated by the reaction mixture, which inter alia is to be cooled down in the separator unit (A2).

As already mentioned above in the description of the integrated plant, the methanol purification column can be a single distillation column as well as a combination of two or more distillation columns, which may be interconnected in parallel, in series or be interlaced with each. In the methanol purification distillation, the bottom stream (IVd) from the low boiler column (B2) is separated into at least two streams, namely into a purified methanol stream (V), containing the purified methanol, and a high boiler stream (VI), containing the components with a higher boiling point than methanol. As these high boiling components mainly contain water with a small amount of organic by-products with a boiling point lower than water, it is advantageous for the further handling of the high boiling components to also separate the water from the organic by-products. Therefore, it is preferred to also separate these compounds within the methanol purification distillation and to withdraw a so-called middle boiler stream (VII) containing the organic by-products such as ethanol, propanols and butanols, and a high boiler stream (VI) mainly containing water.

Preferably, the purification distillation is performed in a pressure swing distillation. The typical interconnection of such a pressure swing distillation is described above in the description of the integrated plant.

The person skilled in the art knows how to perform the methanol purification distillation, be it with a single distillation column, a divided wall distillation column or a pressure swing interconnection. In the preferred case of using a pressure swing distillation, the first distillation column is preferably operated at a pressure of 0.6 to 1.0 MPa abs, and the second distillation column operated at a pressure of 0.06 to 0.15 MPa abs, whereas the specific pressure in the first distillation column is higher than that in the second distillation column.

The heat required for the operation of the methanol purification column, irrespective of being a single column or two or more columns, is provided by a heat exchanger, or also called reboiler, which is interconnected with the thermo fluid system (C).

As already mentioned before, methanol plants based on carbon dioxide and hydrogen as raw materials require additional thermal energy for the distillative separation of the methanol containing product stream if methanol with a purity of $\geq$ 98 wt.-% is intended to be produced. This is due to the higher separation performance required for this task. The process of the invention is therefore referred for obtaining in stream (V) purified methanol with a methanol content of 98 to 100 wt.-%. The methanol content in stream (V) is more preferably $\geq$ 98.5 wt.-%, particularly preferably $\geq$ 99 wt.-%, very particularly preferably $\geq$ 99.5 wt.-% and most preferably $\geq$ 99.8 wt.-%. For the sake of completeness, it is mentioned that a methanol purity of $\geq$ 99.85 wt.-% is a worldwide industrial standard purity called "AA grade". Regarding the upper methanol content, it is more preferred at $\leq$ 99.99 wt.-% and particularly preferred at $\leq$ 99.95 wt.-%.

As the streams (III) and (IV) regarding the process based on an integrated plant as shown in Fig. 1 as well as the streams (III), (IVa) and (IVc) regarding the preferred process based on an integrated plant as shown in Fig. 2 contain carbon containing components such as carbon dioxide, carbon monoxide, methanol, methyl formate, diethyl ether and other carbon containing by-products, it may be advantageous to collect these streams, combust them with an oxygen containing gas, preferably with oxygen enriched air or even pure oxygen, in a combustion unit to carbon dioxide, separate the carbon dioxide in a carbon dioxide separation unit, preferably by chemical absorption in a solvent such as an amine, and recycle the so separated carbon dioxide back to the methanol synthesis unit (A). Such a treatment is already described in WO 2021/148,262 to which it is herewith referred to.

Regarding the heat management in the process of the invention, heat generated in the methanol synthesis reactor unit (A1) is removed from it by a thermo fluid, circulating from and to a thermo fluid system (C), and heat required in the methanol recovery unit (B) is provided by a thermo fluid, circulating from and to the thermo fluid system (C), as indicated in the block diagram shown in Fig. 1.

Since it is known that the thermal energy generated in the methanol synthesis reactor unit (A1) is lower than the thermal energy required in the methanol recovery unit (B) for the separation of purified methanol labeled as stream (V), additional thermal energy is to be provided to the process. The core of the invention is to use an electrical heater (EH) or a heat exchanger connected to an electrical heater (EH) to supply the additionally required energy. Therefore, in the process of the invention, 20 to 100% of the difference between the heat required in the methanol recovery unit (B) and the heat generated in the methanol synthesis reactor unit (A1) is supplied to the thermo fluid by an electrical heater or a heat exchanger connected to an electrical heater.

Although a supply of at least 20% of the difference between the heat required in the methanol recovery unit (B) and the heat generated in the methanol synthesis reactor unit (A1) by an electrical heater (EH) or a heat exchanger connected to an electrical heater (EH) is already advantageous in order to reduce the amount of conventional steam with otherwise would be required, it is preferred to supply ≥ 50%, preferably ≥ 75%, more preferably ≥ 90%, particularly preferably ≥ 95%, most particularly preferably ≥ 99% and particularly 100% of the mentioned difference by an electrical heater (EH) or a heat exchanger connected to an electrical heater (EH).

The nature and type of possible electrical heaters as well as that of possible heat exchangers connected to an electrical heater, and their interconnection within the thermo fluid system (C) has already been described above in the description of the integrated plant. The source at which and how the electricity required for the operation of the electrical heater is generated is basically not restricted, so that also e.g. electricity generated by conventional power plants can be used for the process of invention, but it is favorable to use environmental friendly electricity generated by renewable sources and thus without producing climate-damaging emissions, such as electricity generated by wind, solar or geothermal power.

The thermo fluid used in the process of the invention is generally a fluid which is capable of absorbing and dispensing heat, and which has no, or at least a very low corrosion potential towards the equipment being in contact with the thermo fluid. These properties are very well fulfilled for a water based thermo fluid, which is preferred as thermo fluid. Water based means that more than 50 wt.-% of the thermo fluid is water, more preferably ≥ 90 wt.-%, particularly preferable ≥ 95 wt.-%, very particularly preferably ≥ 99 wt.-% and most preferably ≥ 99.5 wt.-% of the thermo fluid is water. To significantly reduce the corrosivity of water, corrosion reducing agents such as ammonia or phosphates are preferably added. Typically, small amounts in the range of ≤ 1000 wt.-ppm and preferably ≤ 100 wt.-ppm or even ≤ 10 wt.-ppm may suffice. Preferably, the pH value of the water based thermo fluid is slightly basic in a range of 8 to 10. The person skilled in the art knows suitable thermo fluids and particularly how to prepare water with corrosion inhibiting agents to be used as thermo fluid.

As already explained above in the description of the integrated plant, water can easily be vaporized under the absorption of vaporization energy, and it can easily be condensed under the release of condensation energy, so that not only thermal energy is absorbed or provided, but also energy from the vaporization or condensation. Hence, the water based thermo fluid is present in the system as liquid water as well as steam, depending on its vapor pressure which at the end is dependent on the pressure and temperature.

In a preferred embodiment of the process of the invention, the thermo fluid system (C) contains a vessel (C1) which functions as a kind of thermo fluid reservoir. A preferred interconnection of vessel (C1) with the methanol synthesis reactor unit (A1), the methanol recovery unit (B) and the electrical heater (EH) or the heat exchanger connected to the electrical heater is shown in Fig. 2. According to that, the thermo fluid

- flows through a valve V1 to the methanol synthesis reactor unit (A1) and from the methanol synthesis reactor unit (A1) back to vessel (C1),
- flows to the methanol recovery unit (B) and from the methanol recovery unit (B) back to vessel (C1), and
- flows to the electrical heater (EH) or the heat exchanger connected to the electrical heater and from the electrical heater (EH) or the heat exchanger connected to the electrical heater via a valve V2 back to vessel (C1).

With these thermo fluid loops, vessel (C1) is supplied with thermal energy from the methanol synthesis reactor unit (A1) and from the electrical heater (EH) or the heat exchanger connected to the electrical heater, and simultaneously provides thermal energy to the methanol recovery unit (B).

In order to avoid pumps for the circulation of the thermo fluid between the vessel (C1) and the methanol synthesis reactor unit (A1) as well as between the vessel (C1) and the electrical heater (EH) or the heat exchanger connected to the electrical heater, the vessel (C1) is preferably located at a height

- above the thermo fluid outlet of the methanol synthesis reactor within the methanol synthesis reactor unit (A1) from which the thermo fluid flows out, and
- also above the thermo fluid outlet of the electrical heater (EH) or the heat exchanger connected to the electrical heater.

This arrangement enables the use of the thermosyphon effect, where the thermo fluid having a lower temperature, and therefore a higher density, flows downwards by gravity, and the thermo fluid having a higher temperature, and therefore a lower density, flows upwards.

More preferably and in view of the interconnection for starting-up the integrated plant,

- the vessel (C1) is located at a height above the thermo fluid outlet of the methanol synthesis reactor within the methanol synthesis reactor unit (A1) from which the thermo fluid flows out, and
- the electrical heater (EH) or the heat exchanger connected to the electrical heater is located at a height below the

thermo fluid inlet of the methanol synthesis reactor within the methanol synthesis reactor unit (A1) from which the thermo fluid flows in.

As already explained above in the description of the integrated plant, the thermo fluid supplied to the methanol synthesis unit (A) has favorably a higher temperature than the thermo fluid supplied to the heat exchanger of the methanol purification column. Consequently, also the pressure of the thermo fluid supplied to the methanol synthesis unit (A) is higher than the pressure of the thermo fluid supplied to the heat exchanger of the methanol purification column. Therefore, the pressure of the thermo fluid in vessel (C1) is preferably adapted to the thermal conditions given by the methanol synthesis reactor unit (A1) so that a mixture of liquid and steam exists in vessel (C1), and the thermo fluid leaving vessel (C1) to the methanol recovery unit (B) depressurized to a pressure which is adapted to the thermal conditions required for the heating up of the bottoms of the methanol purification column, and the recycle stream of the thermo fluid pressurized before entering vessel (C1) again. In such a preferred process, the pressure in vessel (C1) is typically 1.5 to 5.0 MPa abs and the pressure of the thermo fluid for heating up the bottoms of the methanol purification column 0.4 to 1.0 MPa abs, whereas the pressure in vessel (C1) is preferably about 1.1 to 4.0 MPa higher than the pressure of the thermo fluid for heating up the bottoms of the methanol purification column.

For practical reasons, the thermo fluid is preferably depressurized into a so-called steam and condensate system (C2) as a further unit. A block diagram showing such a preferred unit is given in Fig. 3. The steam and condensate system (C2) is in the easiest way a container having inlets and outlets to which the thermo fluid of vessel (C1) is depressurized and from which the flowback to vessel (C1) is withdrawn and pressurized. Preferably, the thermo fluid is not only depressurized by entering the steam and condensate system (C2), but also de-superheated to a temperature which is 2 to 10 K above the dewpoint of the thermo fluid. However, it typically also functions as a unit for the exchange of thermo fluid as it is favorable to preferably continuously remove a small part of the thermo fluid in order to avoid an accumulation of salts and other impurities. Consequently, fresh thermo fluid has to be supplied. Therefore, in a preferred process of the invention, condensed thermo fluid is withdrawn as blow down, gaseous thermo fluid withdrawn as flash steam, and liquid thermo fluid supplied as boiler feed water.

The inventive use of an electrical heater or a heat exchanger connected to the electrical heater as source for the provision of thermal energy has not only the advantage that environmental friendly energy can be used for the provision of the additional thermal energy required, but also the advantage that the process can quickly and easily be adapted on fluctuations of the availability of the raw materials carbon dioxide and hydrogen, which might occasionally occur by using renewable sources. Electrical heaters are known to be adapted very quickly.

Simulations showed that the methanol synthesis as well as the distillation columns in the methanol recovery unit can easily be brought down from 100% load, meaning 100% of the capacity for which the integrated plant is designed for in accordance with recognized engineering practice, to around 60% load, with an approximately linear decrease of the required electrical energy, or in other words with a nearly constant electrical energy per ton of methanol. A further reduction of the load from 60% to 30% is also easily possible, but with the consequence that the reflux in the distillation columns has to be increased in order to maintain the hydraulic conditions. This causes an increase of the heat required for heating up the bottoms, so that also the required electrical energy per ton of methanol increases. Even though an increased amount of electrical energy is required in case of a drastic reduction of the load, the process of the invention can still be operated even at 30% load.

Furthermore, we have found a process for starting-up an integrated plant as described above for the production of methanol from a carbon dioxide and hydrogen containing stream by

(a) heating a methanol synthesis reactor in the methanol synthesis reactor unit (A1) containing a methanol synthesis catalyst to a temperature of 150 to 260°C by a thermo fluid, circulating from and to a thermo fluid system (C), and

(b) providing a carbon dioxide and hydrogen containing stream (I) at a pressure of 3 to 10 MPa abs to the methanol synthesis reactor in the methanol synthesis reactor unit (A1),

wherein 20 to 100% of the required heat is supplied to the thermo fluid by an electrical heater (EH) or a heat exchanger connected to an electrical heater (EH).

There are mainly three typical reasons at which a start-up of the integrated plant is performed.

One reason is the start-up of the integrated plant after the methanol synthesis catalyst was exchanged by a new one. Since a newly installed methanol synthesis catalyst typically needs to be reduced first, it has to be treated with hydrogen under an elevated temperature, typically at ≥ 200°C, for its conditioning. Such a procedure requires the heating of the reactor and the maintaining of the heat for the reduction period. Therefore, in a preferred process for starting-up the integrated plant, the methanol synthesis catalyst is conditioned in step (a) in the presence of hydrogen.

A second reason is the start-up of the integrated plant after it has been shut-down, e.g. after maintenance, after a rebuilding or modification, after a time of intended non-use, or after a disruption. Last but not least, a third reason

is the start-up of the integrated plant after it has been kept in a stand-by mode. Stand-by mode means that the methanol synthesis catalyst is kept warm to have the chance to directly start the plant. Therefore, in another preferred process for starting-up the integrated plant, the methanol synthesis catalyst is heated in step (a) in the presence of hydrogen, nitrogen or a mixture thereof.

In a preferred embodiment of the process of the invention, the process of starting-up is performed in an integrated plant having interconnections as shown in the block diagrams shown in Fig. 2 or Fig. 3. The tricky interconnection between the vessel (C1), the electrical heater (EH) or the heat exchanger connected to the electrical heater, and the methanol synthesis reactor unit (A1) regarding the flow of the thermo fluid, and particularly the arrangement of the valves V1, V2 and V2 enable a very advantageous operation of the start-up process. In this preferred process,

- the thermo fluid is circulated during the heating in step (a) from vessel (C1) to the electrical heater (EH) or the heat exchanger connected to the electrical heater, then via valve V3 to the methanol synthesis reactor unit (A1), and back to vessel (C1), and then
- for starting-up the conversion, valve V3 is closed and the thermo fluid is circulated from vessel (C1) via valve V1 to the methanol synthesis reactor unit (A1), and back to vessel (C1).

As soon as the thermo fluid in vessel (C1) needs to be heated up after the process for the production of methanol has been started-up, due to the demand of thermal energy for the performance of the methanol purification distillation, the thermo fluid can easily be heated up in the electrical heater (EC) or the heat exchanger connected to the electrical heater by circulating from vessel (C1) to the electrical heater (EH) or the heat exchanger connected to the electrical heater and back via valve V2 to vessel (C1).

If the arrangement of the vessel (C1), the methanol synthesis reactor unit (A1) and the electrical heater (EH) or the heat exchanger connected to the electrical heater is performed in a way that

- the vessel (C1) is located at a height above the thermo fluid outlet of the methanol synthesis reactor within the methanol synthesis reactor unit (A1) from which the thermo fluid flows out, and
- the electrical heater (EH) or the heat exchanger connected to the electrical heater is located at a height below the thermo fluid inlet of the methanol synthesis reactor within the methanol synthesis reactor unit (A1) from which the thermo fluid flows in,

  the thermosyphon effect can be used, as already describe above, which enables the heat induced circulation of the thermo fluid without requiring pumps.

The core of the invention is to use an electrical heater or a heat exchanger connected to an electrical heater to supply the additionally required energy. This does also apply for the starting-up of the plant. Therefore, in the process of the invention, 20 to 100% of the heat required for the heating of the methanol synthesis reactor in the methanol synthesis reactor unit (A1) is supplied to the thermo fluid by an electrical heater (EH) or a heat exchanger connected to an electrical heater (EH). Although a supply of at least 20% of the required heat by an electrical heater (EH) or a heat exchanger connected to an electrical heater (EH) is already advantageous in order to reduce the amount of conventional steam with otherwise would be required, it is preferred to supply ≥ 50%, preferably ≥ 75%, more preferably ≥ 90%, particularly preferably ≥ 95%, most particularly preferably ≥ 99% and particularly 100% of the required heat by an electrical heater (EH) or a heat exchanger connected to an electrical heater (EH).

A preferred embodiment of an integrated plant according to the invention is shown in Fig. 4 and 5. The labels and short names therein have the following meaning (alphabetical order).

| | |
|---|---|
| C01 | feed gas compressor |
| C02 | recycle gas compressor |
| D01 | low boiler column |
| D02 | high pressure column for the purification of methanol |
| D03 | low pressure column for the purification of methanol |
| E02 | gas/gas heat exchanger |
| E03 | heat exchanger |
| E04 | cooler |
| E05 | reboiler |
| E06 | cooler |
| E07 | reboiler |
| E08 | heat exchanger |
| E09 | cooler |
| EH01 | electrical heater |

| | |
|---|---|
| F01 | steam drum |
| F02 | gas/liquid separator |
| F03 | gas/liquid separator |
| F04 | gas/liquid separator |
| F05 | low pressure vessel |
| R01 | methanol synthesis reactor |
| SCS | steam and condensate system |
| V1 | valve |
| V2 | valve |
| V3 | valve |

(I) carbon dioxide and hydrogen containing stream

(IIIa) reaction mixture (containing methanol, water, carbon dioxide, carbon monoxide and hydrogen)

(IIIb) carbon dioxide, carbon monoxide and hydrogen containing recycle stream

(III) carbon dioxide, carbon monoxide and hydrogen containing stream (also called purge gas)

(II) methanol and water enriched raw methanol stream (also called raw methanol)

(IVa) expansion gas

(IVb) degassed raw methanol stream

(IVc) low boiler stream from low boiler column D01

(IVd) bottom stream from low boiler column D01

(V) purified methanol stream

(VI) high boiler stream

(VII) middle boiler stream

(X) boiler feed water (additional water to compensate the losses of water)

(XI) blow-down (condensed steam for discharging impurities)

(XII) steam to D05 and D07

(XIII) steam condensates from D05 and D07

(XIV) steam for boiler feed water degassing

| | |
|---|---|
| $CO_2$ | carbon dioxide stream |
| $H_2$ | hydrogen stream |
| bottom D01 | cross reference indicating the type of the stream applied |
| cooling | cooler operated with a cooling medium |
| steam | steam supplied from SCS via stream (XII) to E05 and E07 and being recycled back to SCS as steam condensates |

The heat exchanger E03 is drawn twice. Once in dotted lines in Fig. 4 and once in solid lines in Fig. 5. This indicates that the gaseous stream released in F02 is used via E03 to heat up the bottom stream of D01 before is re-enters the column D01. The gaseous stream of F02 is hereby cooled down and fed to F03.

First, the interconnection together with the operation mode of the integrated plant is described in more detail.

A mixture of the carbon dioxide and hydrogen feed stream, which is also called make-up gas stream and indicated as stream (I), is compressed to synthesis pressure by the feed gas compressor C01 and mixed with the recycle-gas stream (IIIb). The desired hydrogen/carbon dioxide stoichiometry in the methanol synthesis reactor (or shortly reactor) is adjusted by the amount of hydrogen feed to be added. The mixed feed and recycle-gas stream is then pre-heating in the gas/gas heat exchanger E02 to the reactor inlet temperature and fed to the reactor R01. Preferably, reactor R01 is a quasi-isothermal water cooled reactor. Reactor R01 contains the methanol synthesis catalyst. In reactor R01, methanol is formed from hydrogen, carbon dioxide (according to reaction equation (1)) and carbon monoxide (according to reaction equation (1)), whereas carbon monoxide is formed by the reverse water-gas shift (according to reaction equation (3)). Generally, the content of carbon monoxide is lower than that of carbon dioxide. Beside methanol and water, also by-products in minor amounts, such as dimethyl ether, ethanol, methyl formate, propanols, butanols or, in case of a very active catalyst, even methane are also formed. To avoid a high temperature increase due to the exothermic reaction, the catalyst is cooled by boiling water in a thermosyphon system. In the thermosyphon system, liquid water flows downwards by gravity (whereas the respective piping is also called downcomer) from the steam drum F01, which is located at a height above the thermo fluid outlet of reactor R01, via valve V01 to the lower thermo fluid inlet of reactor R01. It is then partially evaporated in reactor R01 due to the exothermic reaction, and flows back to the steam drum F01 as a heated-up steam/water-mixture via the thermo fluid outlet of reactor R01 (whereas the respective piping is also called riser).

The reaction mixture (IIIa) leaving the reactor R01 is cooled down against the feed of the reactor R01 in the gas/gas heat exchanger E02. In the heat exchanger E02, a part of the raw methanol of the reaction mixture already condenses and is separated in the gas/liquid-separator F02. The gas-phase out of the gas/liquid-separator F02 is used in the heat exchanger E03 for heating up the bottom stream of the low boiler column D1. A further part of the raw methanol of the gas-phase out stream of the gas/liquid-separator F02 is condensed in E03 and separated in the gas/liquid-separator F03. Last but not least, a further part of the raw methanol of the gas-phase out stream of the gas/liquid-separator F03 is condensed in the cooler E04 and separated in the gas/liquid-separator F04.

The gas-phase out of the last gas/liquid-separator F04 is partly recycled as stream (IIIb) over the recycle gas compressor C02 to the carbon dioxide and hydrogen containing make-up gas stream to cause a high conversion of the reacting components carbon dioxide, hydrogen and carbon monoxide. A small amount of this gas-stream is purged out as stream (III), also called purge-gas, to assure a high partial pressure of the reacting components and to limit the accumulation of inert components in the methanol synthesis loop. Typical inert components are nitrogen and argon, which are introduced with the make-up gas, and, in case of a very active catalyst, also small amounts methane.

The raw methanol condensed out in the gas/liquid-separators F02, F03 and F04 is collected as stream (II), depressurized in the low pressure vessel F05, and guided as stream (IVb) to the low boiler column D01. The released gas (IVa) is separated off as off-gas.

In the low boiler column D01, the raw methanol is further separated from the low boilers, containing dimethyl ether, methyl formate and dissolved gases like carbon dioxide, which are also called light ends, and which are separated off as stream (IVc). Down to the limiting capacity of the distillation section of around 60%, the amount of heat provided from the cooling down of the gaseous stream from the gas/liquid-separator F02 suffices to heat up the bottom stream of the low boiler column D01 via the heat exchanger E03. However, at lower capacities, additional heat is required to keep the minimum hydraulic load for operation. This additional heat is then provided by the reboiler E05, which is supplied with steam from the steam and condensate system SCS. The methanol containing bottom stream of the low boiler column D01 is guided as stream (IVd) to the refining distillation.

In the preferred embodiment shown in Fig. 5, the refining distillation is a two-pressure distillation and comprises two distillation columns, the high pressure column D02 and the low pressure column D03. The first distillation column is the high pressure column D02. Its bottom stream is heated up by the reboiler E07. Reboiler E07 is supplied with steam from the steam and condensate system SCS. Similar to the low boiler column D01, also the heat energy required in the high pressure column D02 increases with decreasing capacities to keep the minimum hydraulic load for operation. The over-head stream of the high pressure column D02 is condensed in heat exchanger E08, which is at once also the reboiler of the low pressure column D03. The condensed over-head stream of the high pressure column D02 is purified methanol. One part of this purified methanol is already withdrawn as stream (V) together with the purified methanol separated off in the low pressure column D03, and the other part recycled back to the high pressure column D02 as reflux. The liquid withdrawal of the high pressure column D02 is fed to the low pressure column D03. In the low pressure column D03, further purified methanol is withdrawn as over-head stream, and one part thereof withdrawn as part of the stream (V), whereas a part of the stream is recycled back as reflux, as usual for distillation columns. A stream of so-called middle boilers, indicated as stream (VII), is withdrawn as a side product from the low pressure column D03, containing typical by-products such as ethanol or higher alcohols like propanols and butanols. From the bottom, a stream of high boilers, indicated as stream (VI), is withdrawn. It contains the water which is formed by the hydrogenation of

carbon dioxide and is also called process water.

The following paragraphs relate to the heat balance and the flow of the thermo fluid of the preferred embodiment.

The used thermo fluid is preferably water based comprising liquid water and steam. As already mentioned above, heat is produced in reactor R01 and transported by the thermo fluid to the steam drum F01. On the other hand, the high pressure column D02 requires thermal energy for heating up the bottom stream. The flow and heat integration of the thermo fluid is handled by a thermo fluid system. In the preferred embodiment shown in Fig. 4, the thermo fluid system comprises the steam drum F01 as a thermo fluid vessel, which is interconnected with a steam and condensate system SCS and an electrical heater EH01. The steam and condensate system SCS is an interconnected system with inlets and outlets for the thermo fluid. The steam drum F01 contains a pressurized mixture of liquid water and steam, preferably at a pressure in the range of 1.5 to 5.0 MPa abs. As already mentioned above, the liquid water flows downwards by gravity from the steam drum F01 via valve V01 to the lower thermo fluid inlet of reactor R01, and after being heated up therein as a steam/water-mixture via the thermo fluid outlet of reactor R01 back to the steam drum F01. Generated pressurized steam is withdrawn from the steam drum F01 and guided to the steam and condensate system SCS, whereas it is thereby depressurized to a lower pressure in the range of 0.4 to 1.0 MPa abs and de-superheated to a temperature which is 2 to 10 K above the dewpoint of the water based thermo fluid. Such depressurized and de-superheated steam is also called low-pressure steam. Low-pressure-steam of the steam and condensate system SCS is guided via stream (XII) to the reboiler E05 and the heat exchanger E07 to heat up the bottom streams of the two distillation columns D01 (if required) and D02, and recycled back as condensate to the steam and condensate system SCS as stream (XIII). Since steam is guided from the steam drum F01 to the steam and condensate system SCS as mentioned above, condensates are recycle back from the steam and condensate system SCS to the steam drum F01 to close the loop.

To avoid the accumulation of impurities in the thermo fluid, such as salts, boiler feed water is fed as stream (X) to the steam and condensate system SCS and condensed steam withdrawn as stream (XI) from the steam drum F01 as a blow-down. Last but not least, steam for boiler feed water degassing is withdrawn from the steam and condensate system SCS as stream (XIV).

As already mentioned before, the operation of the integrated plant for the production of methanol from carbon dioxide and hydrogen requires additional heat to operate the methanol recovery system to be able to produce so-called purified methanol with a methanol content of 98 to 100 wt.-%. In the preferred embodiment shown in Fig. 4, this additional heat is provided by an electrical heater EH01 which is interconnected with the heat drum F01. Liquid water flows downwards by gravity from the steam drum F01 to the electrical heater EH01, which is located at a height below the thermo fluid inlet of reactor R01. It is then partially evaporated and flows back via valve V2 to the steam drum F01 as a heated-up steam/water-mixture. Valve V3 is closed in this operation mode.

Second, the start-up of the integrated plant is described in more detail.

Start-ups are normally performed after the change of the methanol synthesis catalyst and after shut-downs of the plant. Since a newly installed methanol synthesis catalyst typically needs to be reduced first, it has to be treated with hydrogen under an elevated temperature, typically above 200°C. Such procedure requires the heating of the reactor and the maintaining of the heat for the reduction period. However, even if the methanol synthesis catalyst was not changed, the temperature in the reactor R01 might be below the reaction temperature, e.g. after a disruption or a shut-down, and therefore the reactor needs to be heated up. A third case would be a stand-by operation, in which it is useful to keep the catalyst warm to have the chance to directly start the plant. In such cases, the required heat is provided by the electrical heater. At the start-up mode, valves V2 and V1 are closed or at least partially closed, and valve V3 open or at least partially open to enable the flow of the thermo fluid heated by the electrical heater EH01 via valve 3 through the reactor R01 and back to the heat drum F01.

The integrated plant of the invention enables the production of methanol in a high yield and with a purity of ≥ 98 wt.-% from a carbon dioxide and hydrogen containing stream without requiring external steam to close the gap between the heat energy generated by the conversion of carbon dioxide and hydrogen, and the heat energy required for the distillation of the raw methanol stream to purified methanol. Due to the internal generation of steam by an electrical heater or a heat exchanger connected to an electrical heater, the integrated plant can compensate its demand of additional heat energy during the production of methanol in an environmental friendly manner by using electrical energy generated by a renewable energy source. Furthermore, the amount of energy to be generated in the electrical heater can easily, flexibly and within a very short response time be adapted to the amount actually required. As the electrical heater has no rotary equipment, it is robust and stable in its operation.

Moreover, the integrated plant can largely use the same apparatuses and interconnections of established methanol synthesis plants customary in the industry and which hitherto have been based on the use of synthesis gas, so that such plants can easily be modified. Moreover, the integrated plant can be easy to operate, even under difficult conditions under which the environmental friendly energy source as well as the carbon dioxide and hydrogen raw materials show considerable fluctuations.

Finally, the integrated plant also enables an easy start-up, e.g. after the installation of a new methanol synthesis catalyst, after a shut-down, or by keeping the temperature in the methanol synthesis reactor during stand-by, by easily

providing the thermal energy by the electrical heater, too.

Examples

The examples were simulated with Aspen Plus™ V11 simulation software. Basis for the calculation were two different interconnections, one with an inventive interconnection for the inventive process, and the other with a comparative interconnection for a comparative process based on the state of the art. The two interconnections only differ in the thermo fluid loop and its energetic treatment.

Interconnection 1 (inventive)

Interconnection 1 is an inventive interconnection as shown in Fig. 4 and 5. The interconnection, including their labels and short names as well as their operation was already described beforehand as a preferred embodiment in the above description. Therefore, it is only emphasized that the inventive interconnection 1 is specified by an electrical heater EH01, the valves V1 to V3, an appropriate interconnection of these devices, and the absence of an input of external steam to the steam and condensate system SCS.

Interconnection 2 (comparative)

Interconnection 2 is a comparative interconnection based on the state of the art process, and shown in Fig. 6 to 7. Since interconnection 2 only differs from interconnection 1 in the interconnection of the thermo fluid loop and its energetic treatment, it is also referred to the beforehand description and the differences emphasized.

Instead of an electrical heater for providing the required additional heat, along with an appropriate piping and instrumentation of it, interconnection 2 contains an additional input to the steam and condensate system SCS for the provision of external steam (X) as additional thermo source.

Example 1 (inventive)

Example 1 shows a simulation of an inventive production of methanol from carbon dioxide and hydrogen based on interconnection 1 and a predetermined load of 100% relating to the reactor and the distillation columns, including the hydraulic load of the distillation columns. The predetermined process parameters on which the simulation was based are shown in table 1. Based on a typical conversion rate and selectivity of a typical methanol synthesis catalyst, the composition of the streams (I) to (VII) was calculated and is summarized in table 2. The per-pass conversion of $CO_2$ in the reactor was 39.4%, and the per-pass conversion of CO -0.11 % (minus 0.11). The negative value is based on the reverse water-gas shift reaction, which generates in the total balance a small amount of CO from $CO_2$. Table 6 summarizes the energy balance of the thermo fluid stream. Since the reactor cooling steam pressure is 3.34 MPa abs and its temperature 240°C, whereas the steam used for the distillation has only a pressure of 0.7 MPa abs and a temperature of 170°C due to the depressurization of the steam between F01 and E05 and E07, respectively, the amount of steam from the methanol synthesis reactor to the steam drum F01 was recalculated as it would have been steam at 0.7 MPa abs and 170°C. Therefore, both steams can be compared as they carry the same energy per unit, i.e. per kg steam per $t_{MeOH}$.

The methanol synthesis was predetermined to be operated at 7.85 MPa abs and at a temperature of 230°C at the reactor inlet. The reactor was cooled with the aqueous thermo fluid at 3.34 MPa abs under generation of steam which effected a temperature of 240°C at the outlet. The reactor outlet stream showed an inert content of 3 vol.-% (calculated as $N_2$). The stoichiometric number S was 4.6. Hence, the synthesis worked at a high partial pressure of the reactants and a high $H_2$ excess, which is beneficial for the catalyst activity and lifetime. The catalyst was charged with a gas hourly space velocity GHSV of 5500 1/h (specified in $Nm^3_{feedgas}$ / ($m^3_{Cata\text{-}lyst}$ * time [h])) and the recycle loop was running with a recycle ratio (RR) of 2.96 (specified in $Nm^3_{recyclegas}$ / $Nm^3_{feedgas}$). For the distillation, a slightly superheated steam (superheated to 10 K above the dewpoint) with a pressure of 0.7 MPa abs and a temperature of 170°C was used.

The methanol synthesis reactor R01 generated a waste heat of 777 $kg_{steam}/t_{MeoH}$ (recalculated as it would have been steam at 0.7 MPa abs and 170°C). The low boiler column D01 needed no additional steam because the waste heat from the raw methanol cooling section was sufficient. However, the purified methanol distillation in the heat integrated columns D02 and D03 required a heat of 951 $kg_{steam}/t_{MeOH}$, which is well above the heat generated in the methanol synthesis reactor R01. In addition to this, further 24 $kg_{steam}/t_{MeoH}$ are consumed by the boiler feed water degassing via stream (XIV). This caused a lack of heat of 198 $kg_{steam}/t_{MeOH}$, which was balanced by the electric heater EH01. For this steam production, the electric heater EH01 needed an electrical energy of 120 $kW/t_{MeOH}$.

To sum up, at a load of 100%, around 79.7% of the steam needed for the production of purified methanol is produced by the heat of reaction in the methanol synthesis reactor R01, and around 20.3% of the total heat is additionally

required, or in other words, 120 $kW/t_{MeOH}$ have to be additionally provided. According to the invention, this provision was performed by the electric heater EH01, which was interconnected with the steam drum F01 and provided the additional thermal energy to F01 for its further distribution via the steam and condensate system SCS to the reboiler E07.

Example 2 (inventive)

Example 2 shows a simulation of an inventive production of methanol from carbon dioxide and hydrogen based on interconnection 1 and a predetermined load of 60% relating to the reactor and the distillation columns, including the hydraulic load of the distillation columns. The predetermined process parameters on which the simulation was based are shown in table 1. Based on a typical conversion rate and selectivity of a typical methanol synthesis catalyst, the composition of the streams (I) to (VII) was calculated and is summarized in table 3. The per-pass conversion of $CO_2$ in the reactor was 42.3%, and the per-pass conversion of CO -0.12% (minus 0.12). The negative value is based on the reverse water-gas shift reaction, which generates in the total balance a small amount of CO from $CO_2$. Table 6 summarizes the energy balance of the thermo fluid stream. As already explained in example 1, the reactor cooling steam which actually had a pressure of 3.34 MPa abs and a temperature of 240°C was recalculated for comparison as it would have been a steam with 0.7 MPa abs and 170°C. Therefore, both steams can be compared as they carry the same energy per unit, i.e. per kg steam per $t_{MeOH}$.

The methanol synthesis was predetermined to be operated at 7.85 MPa abs and at a temperature of 230°C at the reactor inlet. The reactor was cooled with the aqueous thermo fluid at 3.34 MPa abs under generation of steam which effected a temperature of 240°C at the outlet.

The reactor outlet stream showed an inert content of 2.9 vol.-% (calculated as $N_2$). The stoichiometric number S was 4.6. Hence, the synthesis worked at a high partial pressure of the reactants and a high $H_2$ excess, which is beneficial for the catalyst activity and lifetime. The catalyst was charged with a gas hourly space velocity GHSV of 3000 1/h (specified in $Nm^3_{feedgas}$ / ($m^3_{Cata\text{-}lyst}$ * time [h])), which reflects the lower load, and the recycle loop was running with a recycle ratio (RR) of 2.64 (specified in $Nm^3_{recyciegas}$ / $Nm^3_{feedgas}$). For the distillation, a slightly superheated steam (superheated to 10 K above the dewpoint) with a pressure of 0.7 MPa abs and a temperature of 170°C was used.

The methanol synthesis reactor R01 generated a waste heat of 793 $kg_{steam}/t_{MeoH}$ (recalculated as it would have been steam at 0.7 MPa abs and 170°C). The generated waste heat is higher than that mentioned in example 1 because due to a higher residence time, the per-pas-conversion was higher and therefore also the generated waste heat. The low boiler column D01 needed no additional steam because the waste heat from the raw methanol cooling section was still sufficient. However, the purified methanol distillation in the heat integrated columns D02 and D03 required a heat of 951 $kg_{steam}/t_{MeOH}$, which is well above the heat generated in the methanol synthesis reactor R01. In addition to this, further 35 $kg_{steam}/t_{MeoH}$ are consumed by the boiler feed water degassing via stream (XIV). This caused a lack of heat of 193 $kg_{steam}/t_{MeOH}$, which was balanced by the electric heater EH01. For this steam production, the electric heater EH01 needed an electrical energy of 116 $kW/t_{MeOH}$.

To sum up, at a load of 60%, around 80.4% of the steam needed for the production of purified methanol is produced by the heat of reaction in the methanol synthesis reactor R01, and around 19.6% of the total heat is additionally required, or in other words, 116 $kW/t_{MeOH}$ have to be additionally provided. According to the invention, this provision was performed by the electric heater EH01, which was interconnected with the steam drum F01 and provided the additional thermal energy to F01 for its further distribution via the steam and condensate system SCS to the reboiler E07.

Example 3 (inventive)

Example 3 shows a simulation of an inventive production of methanol from carbon dioxide and hydrogen based on interconnection 1 and a predetermined load of 30% relating to the reactor and the distillation columns, but with the peculiarity that the hydraulic load of the distillation columns was maintained at 60% by an increased reflux ratio, since a hydraulic load of significantly less than 60% would have caused a break-down of the distillation. The predetermined process parameters on which the simulation was based are shown in table 1. Based on a typical conversion rate and selectivity of a typical methanol synthesis catalyst, the composition of the streams (I) to (VII) was calculated and is summarized in table 4. The per-pass conversion of $CO_2$ in the reactor was 44.9%, and the per-pass conversion of CO -0.14% (minus 0.14). The negative value is based on the reverse water-gas shift reaction, which generates in the total balance a small amount of CO from $CO_2$. Table 6 summarizes the energy balance of the thermo fluid stream. As already explained in example 1, the reactor cooling steam which actually had a pressure of 3.34 MPa abs and a temperature of 240°C was recalculated for comparison as it would have been a steam with 0.7 MPa abs and 170°C. Therefore, both steams can be compared as they carry the same energy per unit, i.e. per kg steam per $t_{MeOH}$.

The methanol synthesis was predetermined to be operated at 7.85 MPa abs and at a temperature of 230°C at the reactor inlet. The reactor was cooled with the aqueous thermo fluid at 3.34 MPa abs under generation of steam which effected a temperature of 240°C at the outlet. The reactor outlet stream showed an inert content of 3.0 vol.-%

(calculated as $N_2$). The stoichiometric number S was 4.6. Hence, the synthesis worked at a high partial pressure of the reactants and a high $H_2$ excess, which is beneficial for the catalyst activity and lifetime. The catalyst was charged with a gas hourly space velocity GHSV of 1400 1/h (specified in $Nm^3_{feedgas}$ / ($m^3_{Catalyst}$ * time [h])), which reflects the low load, and the recycle loop was running with a recycle ratio (RR) of 2.39 (specified in $Nm^3_{recyclegas}$ / $Nm^3_{feedgas}$). For the distillation, a slightly superheated steam (superheated to 10 K above the dewpoint) with a pressure of 0.7 MPa abs and a temperature of 170°C was used.

The methanol synthesis reactor R01 generated a waste heat of 808 $kg_{steam}/t_{MeoH}$ (recalculated as it would have been steam at 0.7 MPa abs and 170°C). The generated waste heat is again higher than that mentioned in example 2 because the residence time was further increased and therefore also the generated waste heat caused by the higher per-pass-conversion. In contract to examples 1 and 2, the low boiler column D01 needed 119 $kg_{steam}/t_{MeoH}$ additional steam to keep the hydraulic load of 60% running. The purified methanol distillation in the heat integrated columns D02 and D03 also required with 1832 $kg_{steam}/t_{MeoH}$ a significantly increased amount of heat due to the provision of a hydraulic load of 60%. In addition to this, further 55 $kg_{steam}/t_{MeoH}$ are consumed by the boiler feed water degassing via stream (XIV). This caused a significant lack of heat of 1198 $kg_{steam}/t_{MeOH}$, which was balanced by the electric heater EH01. For this steam production, the electric heater EH01 needed an electrical energy of 723 $kW/t_{MeOH}$.

To sum up, at a load of 30%, but considering a hydraulic load of 60%, only around 40.3% of the steam needed for the production of purified methanol is produced by the heat of reaction in the methanol synthesis reactor R01, and around 59.7% of the total heat is additionally required, or in other words, 1198 $kW/t_{MeOH}$ have to be additionally provided. According to the invention, this provision was performed by the electric heater EH01, which was interconnected with the steam drum F01 and provided the additional thermal energy to F01 for its further distribution via the steam and condensate system SCS to the reboilers E05 and E07.

Example 4 (comparative)

Example 4 shows a simulation of a conventional production of methanol from carbon dioxide and hydrogen based on interconnection 2 and a predetermined load of 100% relating to the reactor and the distillation columns, including the hydraulic load of the distillation columns. The predetermined process parameters on which the simulation was based are shown in table 1. Based on a typical conversion rate and selectivity of a typical methanol synthesis catalyst, the composition of the streams (I) to (VII) was calculated and is summarized in table 5. The per-pass conversion of $CO_2$ in the reactor was 39.4%, and the per-pass conversion of CO -0.11% (minus 0.11). The negative value is based on the reverse water-gas shift reaction, which generates in the total balance a small amount of CO from $CO_2$. Table 6 summarizes the energy balance of the thermo fluid stream. As already explained in example 1, the reactor cooling steam which actually had a pressure of 3.34 MPa abs and a temperature of 240°C was recalculated for comparison as it would have been a steam with 0.7 MPa abs and 170°C. Therefore, both steams can be compared as they carry the same energy per unit, i.e. per kg steam per $t_{MeOH}$.

The methanol synthesis was predetermined to be operated at 7.85 MPa abs and at a temperature of 230°C at the reactor inlet. The reactor was cooled with the aqueous thermo fluid at 3.34 MPa abs under generation of steam which effected a temperature of 240°C at the outlet. The reactor outlet stream showed an inert content of 3 vol.-% (calculated as $N_2$). The stoichiometric number S was 4.6. Hence, the synthesis worked at a high partial pressure of the reactants and a high $H_2$ excess, which is beneficial for the catalyst activity and lifetime. The catalyst was charged with a gas hourly space velocity GHSV of 5500 1/h (specified in $Nm^3_{feedgas}$ / ($m^3_{Cata\text{-}lyst}$ * time [h])) and the recycle loop was running with a recycle ratio (RR) of 2.96 (specified in $NM^3_{recyclegas}$ / $Nm^3_{feedgas}$). For the distillation, a slightly superheated steam (superheated to 10 K above the dewpoint) with a pressure of 0.7 MPa abs and a temperature of 170°C was used.

As the synthesis of the methanol as well as its further processing to purified methanol was exactly the same as in example 1, and only the manner of the provision of the additional energy differed, the predetermined process parameters remained the same as for example 1. Consequently, the methanol synthesis reactor R01 generated a waste heat of 777 $kg_{steam}/t_{MeoH}$ (recalculated as it would have been steam at 0.7 MPa abs and 170°C). The low boiler column D01 needed no additional steam because the waste heat from the raw methanol cooling section was sufficient. However, the purified methanol distillation in the heat integrated columns D02 and D03 required a heat of 951 $kg_{steam}/t_{MeOH}$, which is well above the heat generated in the methanol synthesis reactor R01. In addition to this, further 24 $kg_{steam}/t_{MeoH}$ are consumed by the boiler feed water degassing via stream (XIV). This caused a lack of heat of 198 $kg_{steam}/t_{MeOH}$, which was balanced by the provision of external steam provided by stream (X). The energy of the additionally required steam is 120 $kW/t_{MeOH}$.

Again, also the conventional production of methanol at a load of 100%, around 79.7% of the steam needed for the production of purified methanol is produced by the heat of reaction in the methanol synthesis reactor R01, and around 20.3% of the total heat is additionally required, or in other words 120 $kW/t_{MeOH}$ have to be additionally provided. However, in the conventional production of methanol, this additional heat is provided to the steam and condensate system SCS by external steam (X) from an external thermo source.

Examples 1 to 3 show that the integrated methanol plant can be easily operated without the addition of conventional steam, and thus without additionally emitting considerable amounts of carbon dioxide for the production of the additionally required thermal energy. Even if the external steam (X) in example 4 would be excess heat of other plants, such as chemical plants or power plants, or be produced by other environmentally friendly sources, such as by heat pumps or geothermal power, the methanol plant would not be so flexible in the adjustment of its load and not be so easily started-up.

Table 1 Predetermined process parameters

| | Ex. 1 (inventive) | Ex. 2 (inventive) | Ex. 3 (inventive) | Ex. 4 (comparative) |
|---|---|---|---|---|
| | 100% load | 60% load | 30% load, but 60% hydraulic load in distillation | 100% load |
| Reactor pressure [MPa abs] | 7.85 | 7.85 | 7.85 | 7.85 |
| Reactor inlet temperature [°C] | 230 | 230 | 230 | 230 |
| Reactor cooling temperature [°C] | 240 | 240 | 240 | 240 |
| Reactor cooling steam pressure [MPa abs] | 3.34 | 3.34 | 3.34 | 3.34 |
| Inert components [wt.-%] | 3.0 | 2.9 | 3.0 | 3.0 |
| Stoichiometric number S | 4.6 | 4.6 | 4.6 | 4.6 |
| GHSV [$Nm^3/(m^3$ catalyst ● h] | 5500 | 3000 | 1400 | 5500 |
| Recycle Rate [$Nm^3$ (IIIb)/$Nm^3$ (I)] | 2.96 | 2.64 | 2.39 | 2.96 |
| Steam temperature used for distillation [°C] | 170°C | 170°C | 170°C | 170°C |
| Steam pressure used for distillation [MPa abs] | 0.7 | 0.7 | 0.7 | 0.7 |

Table 2 Stream compositions (example 1)

| Stream | (I) | (II) | (III) | (IIIa) | (IIIb) | (IVa) | (IVb) | (IVc) | (IVd) | (V) | (VII) | (VI) |
|---|---|---|---|---|---|---|---|---|---|---|---|---|
| | | | | | | | | | | | | |
| Amount [unit] | $m^3$ (STP) | kg | $m^3$ (STP) | $m^3$ (STP) | $m^3$ (STP) | kg | kg | kg | kg | kg | kg | kg |
| Amount | 2850 | 565 | 8 | 11939 | 10500 | 12 | 1591 | 16 | 1597 | 1000 | 11 | 586 |
| | | | | | | | | | | | | |
| Composition [unit] | vol.-% | wt.-% | vol.-% | vol.-% | vol.-% | wt.-% | wt.-% | wt.-% | wt.-% | wt.-% | wt.-% | wt.-% |
| $CO_2$ | 25.41 | 2.08 | 10.21 | 9.13 | 10.21 | 78.58 | 0.90 | 88.63 | 0.00 | 0.00 | 0.00 | 0.00 |
| $H_2$ | 74.56 | 0.01 | 84.01 | 73.93 | 84.01 | 14.35 | 0.00 | 0.01 | 0.00 | 0.00 | 0.00 | 0.00 |
| CO | 0.00 | 0.01 | 1.82 | 1.61 | 1.82 | 0.55 | 0.00 | 0.01 | 0.00 | 0.00 | 0.00 | 0.00 |
| Methanol | 0.00 | 62.50 | 0.40 | 6.27 | 0.40 | 3.92 | 63.27 | 0.00 | 63.04 | 100.00 | 56.05 | 0.10 |
| $H_2O$ | 0.00 | 35.26 | 0.04 | 5.97 | 0.04 | 0.33 | 35.71 | 0.87 | 36.95 | 0.00 | 42.32 | 99.90 |
| $N_2$ | 0.03 | 0.02 | 3.51 | 3.09 | 3.51 | 2.04 | 0.00 | 0.08 | 0.00 | 0.00 | 0.00 | 0.00 |
| Dimethyl ether | 0.00 | 0.00 | 0.00 | 0.00 | 0.00 | 0.06 | 0.00 | 0.23 | 0.00 | 0.00 | 0.00 | 0.00 |
| Ethanol | 0.00 | 0.01 | 0.00 | 0.00 | 0.00 | 0.00 | 0.01 | 0.00 | 0.01 | 0.00 | 1.07 | 0.00 |
| Methyl formate | 0.00 | 0.11 | 0.00 | 0.00 | 0.00 | 0.18 | 0.10 | 10.16 | 0.00 | 0.00 | 0.00 | 0.00 |
| Propanols | 0.00 | 0.00 | 0.00 | 0.00 | 0.00 | 0.00 | 0.00 | 0.00 | 0.00 | 0.00 | 0.44 | 0.00 |
| Butanols | 0.00 | 0.00 | 0.00 | 0.00 | 0.00 | 0.00 | 0.00 | 0.00 | 0.00 | 0.00 | 0.12 | 0.00 |

Table 3 Stream compositions (example 2)

| Stream | (I) | (II) | (III) | (IIIa) | (IIIb) | (IVa) | (IVb) | (IVc) | (IVd) | (V) | (VII) | (VI) |
|---|---|---|---|---|---|---|---|---|---|---|---|---|
| | | | | | | | | | | | | |
| Amount [unit] | $m^3$ (STP) | kg | $m^3$ (STP) | $m^3$ (STP) | $m^3$ (STP) | kg | kg | kg | kg | kg | kg | kg |
| Amount | 2850 | 565 | 8 | 10789 | 9350 | 13 | 1591 | 16 | 1612 | 1000 | 7 | 360 |
| | | | | | | | | | | | | |
| Composition [unit] | vol.-% | wt.-% | vol.-% | vol.-% | vol.-% | wt.-% | wt.-% | wt.-% | wt.-% | wt.-% | wt.-% | wt.-% |
| $CO_2$ | 25.40 | 2.05 | 10.16 | 8.97 | 10.16 | 78.24 | 0.88 | 88.46 | 0.00 | 0.00 | 0.00 | 0.00 |
| $H_2$ | 74.57 | 0.01 | 84.37 | 73.17 | 84.37 | 14.75 | 0.00 | 0.01 | 0.00 | 0.00 | 0.00 | 0.00 |
| CO | 0.00 | 0.00 | 1.48 | 1.28 | 1.48 | 0.45 | 0.00 | 0.01 | 0.00 | 0.00 | 0.00 | 0.00 |
| Methanol | 0.00 | 62.52 | 0.39 | 6.90 | 0.39 | 3.92 | 63.28 | 0.00 | 62.47 | 100.00 | 56.19 | 0.10 |
| $H_2O$ | 0.00 | 35.27 | 0.04 | 6.60 | 0.04 | 0.33 | 35.72 | 0.80 | 37.52 | 0.00 | 42.19 | 99.90 |
| $N_2$ | 0.03 | 0.02 | 3.56 | 3.09 | 3.56 | 2.07 | 0.00 | 0.08 | 0.00 | 0.00 | 0.00 | 0.00 |
| Dimethyl ether | 0.00 | 0.00 | 0.00 | 0.00 | 0.00 | 0.06 | 0.00 | 0.24 | 0.00 | 0.00 | 0.00 | 0.00 |
| Ethanol | 0.00 | 0.01 | 0.00 | 0.00 | 0.00 | 0.00 | 0.01 | 0.00 | 0.01 | 0.00 | 1.06 | 0.00 |
| Methyl formate | 0.00 | 0.11 | 0.00 | 0.00 | 0.00 | 0.18 | 0.10 | 10.39 | 0.00 | 0.00 | 0.00 | 0.00 |
| Propanols | 0.00 | 0.00 | 0.00 | 0.00 | 0.00 | 0.00 | 0.00 | 0.00 | 0.00 | 0.00 | 0.44 | 0.00 |
| Butanols | 0.00 | 0.00 | 0.00 | 0.00 | 0.00 | 0.00 | 0.00 | 0.00 | 0.00 | 0.00 | 0.12 | 0.00 |

Table 4 Stream compositions (example 3)

| Stream | (I) | (II) | (III) | (IIIa) | (IIIb) | (IVa) | (IVb) | (IVc) | (IVd) | (V) | (VII) | (VI) |
|---|---|---|---|---|---|---|---|---|---|---|---|---|
| | | | | | | | | | | | | |
| Amount [unit] | $m^3$ (STP) | kg | $m^3$ (STP) | $m^3$ (STP) | $m^3$ (STP) | kg | kg | kg | kg | kg | kg | kg |
| Amount | 2855 | 565 | 7 | 9929 | 8488 | 13 | 1592 | 14 | 1651 | 1000 | 3 | 192 |
| | | | | | | | | | | | | |
| Composition [unit] | vol.-% | wt.-% | vol.-% | vol.-% | vol.-% | wt.-% | wt.-% | wt.-% | wt.-% | wt.-% | wt.-% | wt.-% |
| $CO_2$ | 25.37 | 1.92 | 10.08 | 8.78 | 10.08 | 75.98 | 0.75 | 86.89 | 0.00 | 0.00 | 0.00 | 0.00 |
| $H_2$ | 74.60 | 0.01 | 84.59 | 72.36 | 84.59 | 16.77 | 0.00 | 0.02 | 0.00 | 0.00 | 0.00 | 0.00 |
| CO | 0.00 | 0.00 | 1.17 | 1.00 | 1.17 | 0.40 | 0.00 | 0.01 | 0.00 | 0.00 | 0.00 | 0.00 |
| Methanol | 0.00 | 62.60 | 0.41 | 7.50 | 0.41 | 3.91 | 63.36 | 0.00 | 61.09 | 100.00 | 73.58 | 0.10 |
| $H_2O$ | 0.00 | 35.31 | 0.04 | 7.18 | 0.04 | 0.33 | 35.77 | 0.82 | 38.90 | 0.00 | 24.95 | 99.89 |
| $N_2$ | 0.03 | 0.02 | 3.71 | 3.18 | 3.71 | 2.34 | 0.00 | 0.11 | 0.00 | 0.00 | 0.00 | 0.00 |
| Dimethyl ether | 0.00 | 0.00 | 0.00 | 0.00 | 0.00 | 0.06 | 0.00 | 0.26 | 0.00 | 0.00 | 0.00 | 0.00 |
| Ethanol | 0.00 | 0.01 | 0.00 | 0.00 | 0.00 | 0.00 | 0.01 | 0.00 | 0.01 | 0.00 | 1.03 | 0.00 |
| Methyl formate | 0.00 | 0.11 | 0.00 | 0.00 | 0.00 | 0.19 | 0.10 | 11.89 | 0.00 | 0.00 | 0.00 | 0.00 |
| Propanols | 0.00 | 0.00 | 0.00 | 0.00 | 0.00 | 0.00 | 0.00 | 0.00 | 0.00 | 0.00 | 0.42 | 0.00 |
| Butanols | 0.00 | 0.00 | 0.00 | 0.00 | 0.00 | 0.00 | 0.00 | 0.00 | 0.00 | 0.00 | 0.02 | 0.00 |

Table 5 Stream compositions (example 4)

| Stream | (I) | (II) | (III) | (IIIa) | (IIIb) | (IVa) | (IVb) | (IVc) | (IVd) | (V) | (VII) | (VI) |
|---|---|---|---|---|---|---|---|---|---|---|---|---|
| | | | | | | | | | | | | |
| Amount [unit] | $m^3$ (STP) | kg | $m^3$ (STP) | $m^3$ (STP) | $m^3$ (STP) | kg | kg | kg | kg | kg | kg | kg |
| Amount | 2850 | 565 | 8 | 11939 | 10500 | 12 | 1591 | 16 | 1597 | 1000 | 11 | 586 |
| | | | | | | | | | | | | |
| Composition [unit] | vol.-% | wt.-% | vol.-% | vol.-% | vol.-% | wt.-% | wt.-% | wt.-% | wt.-% | wt.-% | wt.-% | wt.-% |
| $CO_2$ | 25.41 | 2.08 | 10.21 | 9.13 | 10.21 | 78.58 | 0.90 | 88.63 | 0.00 | 0.00 | 0.00 | 0.00 |
| $H_2$ | 74.56 | 0.01 | 84.01 | 73.93 | 84.01 | 14.35 | 0.00 | 0.01 | 0.00 | 0.00 | 0.00 | 0.00 |
| CO | 0.00 | 0.01 | 1.82 | 1.61 | 1.82 | 0.55 | 0.00 | 0.01 | 0.00 | 0.00 | 0.00 | 0.00 |
| Methanol | 0.00 | 62.50 | 0.40 | 6.27 | 0.40 | 3.92 | 63.27 | 0.00 | 63.04 | 100.00 | 56.05 | 0.10 |
| $H_2O$ | 0.00 | 35.26 | 0.04 | 5.97 | 0.04 | 0.33 | 35.71 | 0.87 | 36.95 | 0.00 | 42.32 | 99.90 |
| $N_2$ | 0.03 | 0.02 | 3.51 | 3.09 | 3.51 | 2.04 | 0.00 | 0.08 | 0.00 | 0.00 | 0.00 | 0.00 |
| Dimethyl ether | 0.00 | 0.00 | 0.00 | 0.00 | 0.00 | 0.06 | 0.00 | 0.23 | 0.00 | 0.00 | 0.00 | 0.00 |
| Ethanol | 0.00 | 0.01 | 0.00 | 0.00 | 0.00 | 0.00 | 0.01 | 0.00 | 0.01 | 0.00 | 1.07 | 0.00 |
| Methyl formate | 0.00 | 0.11 | 0.00 | 0.00 | 0.00 | 0.18 | 0.10 | 10.16 | 0.00 | 0.00 | 0.00 | 0.00 |
| Propanols | 0.00 | 0.00 | 0.00 | 0.00 | 0.00 | 0.00 | 0.00 | 0.00 | 0.00 | 0.00 | 0.44 | 0.00 |
| Butanols | 0.00 | 0.00 | 0.00 | 0.00 | 0.00 | 0.00 | 0.00 | 0.00 | 0.00 | 0.00 | 0.12 | 0.00 |

Table 6 Summarized energy balance

| | Ex. 1 (inventive) | Ex. 2 (inventive) | Ex. 3 (inventive) | Ex. 4 (comparative) |
|---|---|---|---|---|
| | 100% load | 60% load | 30% load, but 60% hydraulic load in distillation | 100% load |
| | | | | |
| Steam from reactor R01 [kg/$t_{MeOH}$] | 777 | 793 | 808 | 777 |
| Steam from electrical heater EH01 [kg/$t_{MeOH}$] | 198 | 193 | 1198 | - |
| | | | | |
| Steam for heat exchanger E05 [kg/$t_{MeOH}$] | 0 | 0 | 119 | 0 |
| Steam for heat exchanger E07 [kg/$t_{MeOH}$] | 951 | 951 | 1832 | 951 |
| Steam for boiler feed water degassing [kg/$t_{MeoH}$] | 24 | 35 | 55 | 24 |
| | | | | |
| Lack of energy to be supplied [kW/$t_{MeOH}$] | 120 | 116 | 723 | 120 |
| Nature of the energy to be supplied | electrical heater | electrical heater | electrical heater | external steam |

## Claims

**1.** An integrated plant for the production of methanol from a carbon dioxide and hydrogen containing stream, comprising

(a) a methanol synthesis unit (A) containing a methanol synthesis reactor unit (A1), capable of being cooled or heated by a thermo fluid and having a thermo fluid inlet and outlet, for the conversion of carbon dioxide and hydrogen into a methanol containing reaction mixture, and a separator unit (A2) for its separation into a methanol and water enriched raw methanol stream and a gaseous carbon dioxide, carbon monoxide and hydrogen containing stream, whereas the methanol synthesis unit (A) contains

- an inlet for the inlet of carbon dioxide and hydrogen,
- an outlet for the outlet of the methanol and water enriched raw methanol stream,
- an outlet for the outlet of the gaseous carbon dioxide, carbon monoxide and hydrogen containing stream,
- an inlet for the inlet of a thermo fluid, being interconnected with the thermo fluid inlet of the methanol synthesis reactor unit (A1), and
- an outlet for the outlet of the thermo fluid, being interconnected with the thermo fluid outlet of the methanol synthesis reactor unit (A1),

(b) a methanol recovery unit (B) containing a distillation unit for the separation of the methanol and water enriched raw methanol stream into a stream containing purified methanol, a stream containing components with a lower boiling point than methanol, and a stream containing components with a higher boiling point than methanol, whereas the distillation unit contains a methanol purification column and a heat exchanger for the provision of heat required for the operation of the methanol purification column and having a thermo fluid inlet and outlet, whereas the methanol recovery unit (B) contains

- an inlet for the provision with the methanol and water enriched raw methanol stream, whereas this inlet is interconnected with the outlet for the outlet of the methanol and water enriched raw methanol stream of

the methanol synthesis unit (A),
- an outlet for the outlet of components having a lower boiling point than methanol,
- an outlet for the outlet of the purified methanol,
- an outlet for the outlet of components having a higher boiling point than methanol,
- an inlet for the inlet of a thermo fluid, being interconnected with the inlet of the heat exchanger, and
- an outlet for the outlet of the thermo fluid, being interconnected with the outlet of the heat exchanger,

(c) a thermo fluid system (C) containing a vessel (C1) for the storage and provision of the thermo fluid, whereas the thermo fluid system (C) contains

- an outlet for the outlet of the thermo fluid, whereas this outlet is interconnected with the inlet of the thermo fluid of the methanol synthesis unit (A),
- an inlet for the inlet of the thermo fluid, whereas this inlet is interconnected with the outlet of the thermo fluid of the methanol synthesis unit (A),
- an outlet for the outlet of the thermo fluid, whereas this outlet is interconnected with the inlet of the thermo fluid of the methanol recovery unit (B), and
- an inlet for the inlet of the thermo fluid, whereas this inlet is interconnected with the outlet of the thermo fluid of the methanol recovery unit (B),

whereas the thermo fluid system (C) together with the interconnections to and from the methanol synthesis unit (A) and the methanol recovery unit (B) and their flow paths therein provide a thermo fluid loop, wherein the thermo fluid system (C) further contains an electrical heater (EH) or a heat exchanger connected to an electrical heater (EH).

**2.** The integrated plant according to claim 1, wherein in the methanol recovery unit (B) the distillation unit further contains a low boiler column for the separation of components having a lower boiling point than methanol, being located upstream to the methanol purification column, and a heat exchanger for the provision of heat required for the operation of the low boiler column.

**3.** The integrated plant according to claim 2, wherein in the methanol recovery unit (B) the distillation unit further contains a second methanol purification column being interconnected with the other methanol purification column for their use as a pressure swing distillation.

**4.** The integrated plant according to any of the claims 1 to 3, wherein in the thermo fluid system (C)

- the outlet of the thermo fluid being interconnected with the inlet of the thermo fluid of the methanol recovery unit (B) is interconnected with an outlet of the vessel (C1),
- the inlet of the thermo fluid being interconnected with the outlet of the thermo fluid of the methanol recovery unit (B) is interconnected with an inlet of the vessel (C1),
- the outlet of the thermo fluid being interconnected with the inlet of the thermo fluid of the methanol synthesis unit (A) is interconnected via a valve V1 with an outlet of the vessel (C1),
- the inlet of the thermo fluid being interconnected with the outlet of the thermo fluid of the methanol synthesis unit (A) is interconnected with an inlet of the vessel (C1),
- the electrical heater has an inlet and an outlet of the thermo fluid,
- the inlet for the inlet of the thermo fluid of the electrical heater (EH) or the heat exchanger connected to an electrical heater (EH) is interconnected with an outlet of the vessel (C1),
- the outlet for the outlet of the thermo fluid of the electrical heater (EH) or the heat exchanger connected to an electrical heater (EH) is interconnected through a valve V2 with an inlet of the vessel (C1), and
- the outlet for the outlet of the thermo fluid of the electrical heater (EH) or the heat exchanger connected to an electrical heater (EH) is interconnected through a valve V3 with the outlet of the thermo fluid being interconnected with the inlet of the thermo fluid of the methanol synthesis unit (A).

**5.** The integrated plant according to claim 4, wherein

- the vessel (C1) is located at a height above the thermo fluid outlet of the methanol synthesis reactor within the methanol synthesis reactor unit (A1) from which the thermo fluid flows out, and
- the electrical heater (EH) or the heat exchanger connected to an electrical heater (EH) is located at a height below the thermo fluid inlet of the methanol synthesis reactor within the methanol synthesis reactor unit (A1) from which the thermo fluid flows in.

**6.** A process for the production of methanol by

(a) converting a carbon dioxide and hydrogen containing stream (I) in a methanol synthesis reactor unit (A1) at a temperature of 150 to 300°C and a pressure of 3 to 10 MPa abs in the presence of a methanol synthesis catalyst to a reaction mixture containing methanol, water, carbon dioxide, carbon monoxide and hydrogen, condensing out of said reaction mixture in a gas/liquid separator (A2) a methanol and water enriched raw methanol stream (II) and separating off a gaseous stream (III) comprising carbon dioxide, carbon monoxide and hydrogen,
(b) separating the methanol and water enriched raw methanol stream (II) in a methanol recovery unit (B) by distillation into

- a stream (IV) containing components having a lower boiling point than methanol,
- a stream (V) containing purified methanol, and
- a stream (VI) containing components having a higher boiling point than methanol,

whereas heat generated in the methanol synthesis reactor unit (A1) is removed from it by a thermo fluid, circulating from and to a thermo fluid system (C), and heat required in the methanol recovery unit (B) is provided by a thermo fluid, circulating from and to the thermo fluid system (C), and
wherein 20 to 100% of the difference between the heat required in the methanol recovery unit (B) and the heat generated in the methanol synthesis reactor unit (A1) is supplied to the thermo fluid by an electrical heater (EH) or a heat exchanger connected to an electrical heater (EH).

**7.** The process according to claim 6, wherein the process is performed in an integrated plant according to any of the claims 1 to 5 is used.

**8.** The process according to any of the claims 6 to 7, wherein a copper- and zinc-containing heterogeneous catalyst is used as methanol synthesis catalyst.

**9.** The process according to any of the claims 6 to 8, wherein the purified methanol obtained by stream (V) has a methanol content of 98 to 100 wt.-%.

**10.** The process according to any of the claims 6 to 9, wherein the thermo fluid system (C) contains a vessel (C1)

- from which the thermo fluid flows through a valve V1 to the methanol synthesis reactor unit (A1) and from the methanol synthesis reactor unit (A1) back to vessel (C1),
- from which the thermo fluid flows to the methanol recovery unit (B) and from the methanol recovery unit (B) back to vessel (C1), and
- from which the thermo fluid flows to the electrical heater (EH) or the heat exchanger connected to the electrical heater and from the electrical heater (EH) or the heat exchanger connected to the electrical heater via a valve V2 back to vessel (C1).

**11.** The process according to claim 10, wherein

- the vessel (C1) is located at a height above the thermo fluid outlet of the methanol synthesis reactor within the methanol synthesis reactor unit (A1) from which the thermo fluid flows out, and
- the electrical heater (EH) or the heat exchanger connected to the electrical heater is located at a height below the thermo fluid inlet of the methanol synthesis reactor within the methanol synthesis reactor unit (A1) from which the thermo fluid flows in.

**12.** A process for starting-up an integrated plant according to any of the claims 1 to 5 for the production of methanol from a carbon dioxide and hydrogen containing stream by

(a) heating a methanol synthesis reactor unit (A1) containing a methanol synthesis catalyst to a temperature of 150 to 260°C by a thermo fluid, circulating from and to a thermo fluid system (C), and
(b) providing a carbon dioxide and hydrogen containing stream (I) at a pressure of 3 to 10 MPa abs to the methanol synthesis reactor (A1)

wherein 20 to 100% of the required heat is supplied to the thermo fluid by an electrical heater (EH) or a heat exchanger connected to an electrical heater (EH).

**13.** The process according to claim 12, wherein the methanol synthesis catalyst is conditioned in step (a) in the presence of hydrogen.

**14.** The process according to claim 12, wherein the methanol synthesis catalyst is heated in step (a) in the presence of hydrogen, nitrogen or a mixture thereof.

**15.** The process according to any of the claims 12 to 14, wherein

- the process is performed in an integrated plant according to any of the claims 4 to 5,
- during the heating in step (a), the thermo fluid is circulated from vessel (C1) to the electrical heater (EH) or the heat exchanger connected to the electrical heater, then via valve V3 to the methanol synthesis reactor (A1), and back to vessel (C1), and then
- for starting-up the conversion, valve V3 is closed and the thermo fluid is circulated from vessel (C1) via valve V1 to the methanol synthesis reactor (A1), and back to vessel (C1).

Fig. 1

Block diagram (inventive)

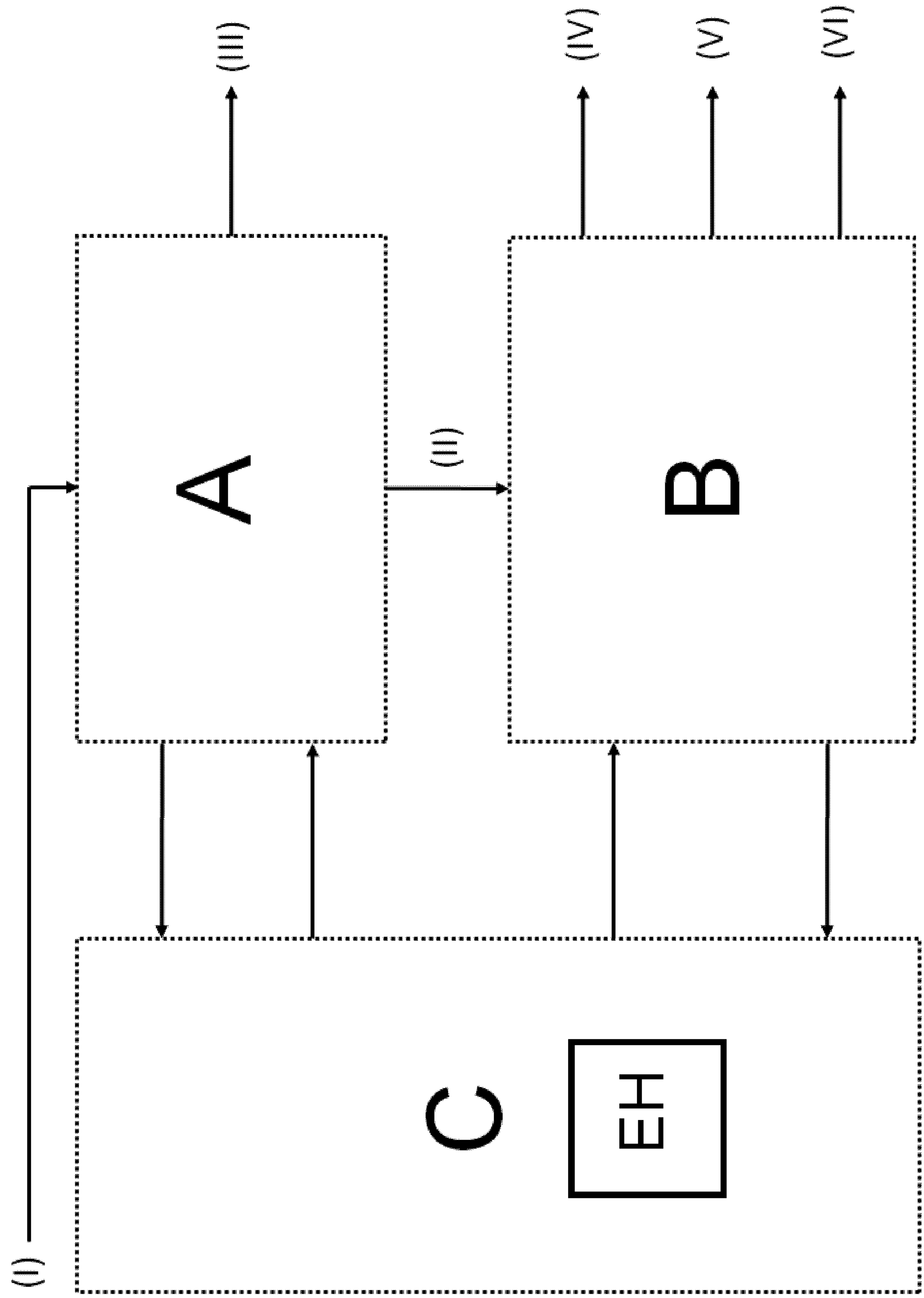

Fig. 2

Block diagram (inventive)

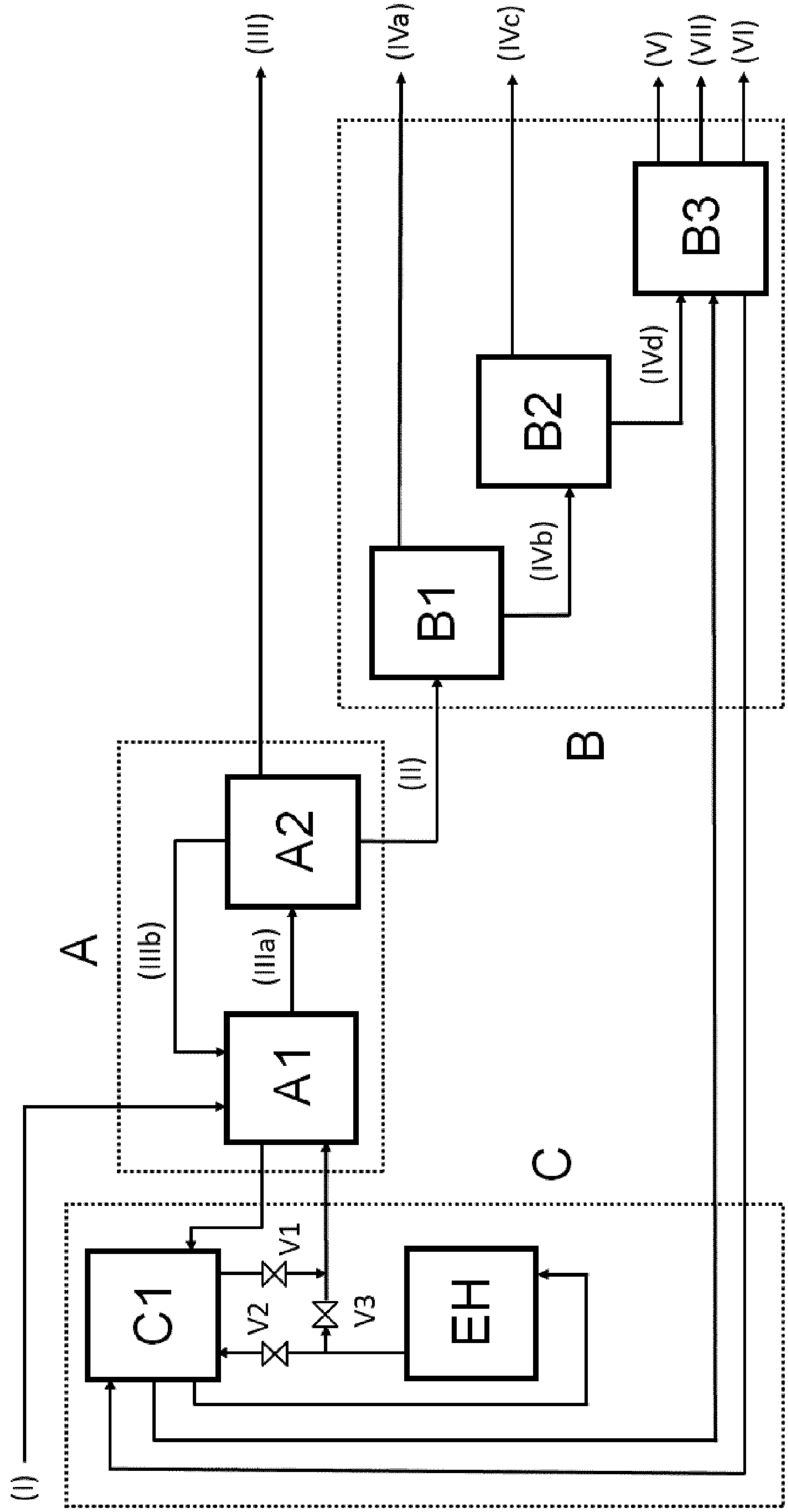

Fig. 3

Block diagram (inventive)

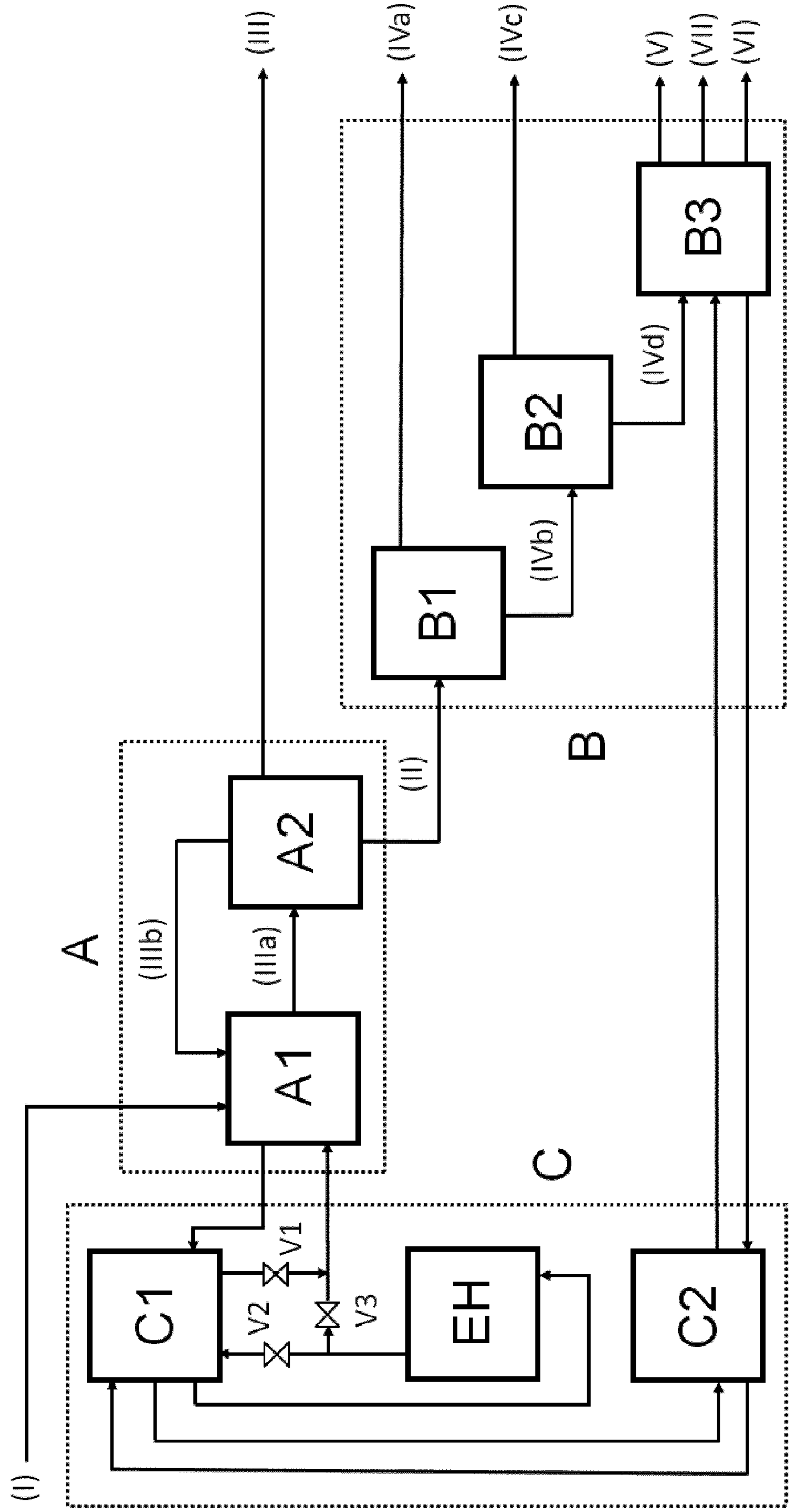

Fig. 4

Process flow diagram, part 1 of 2 (inventive)

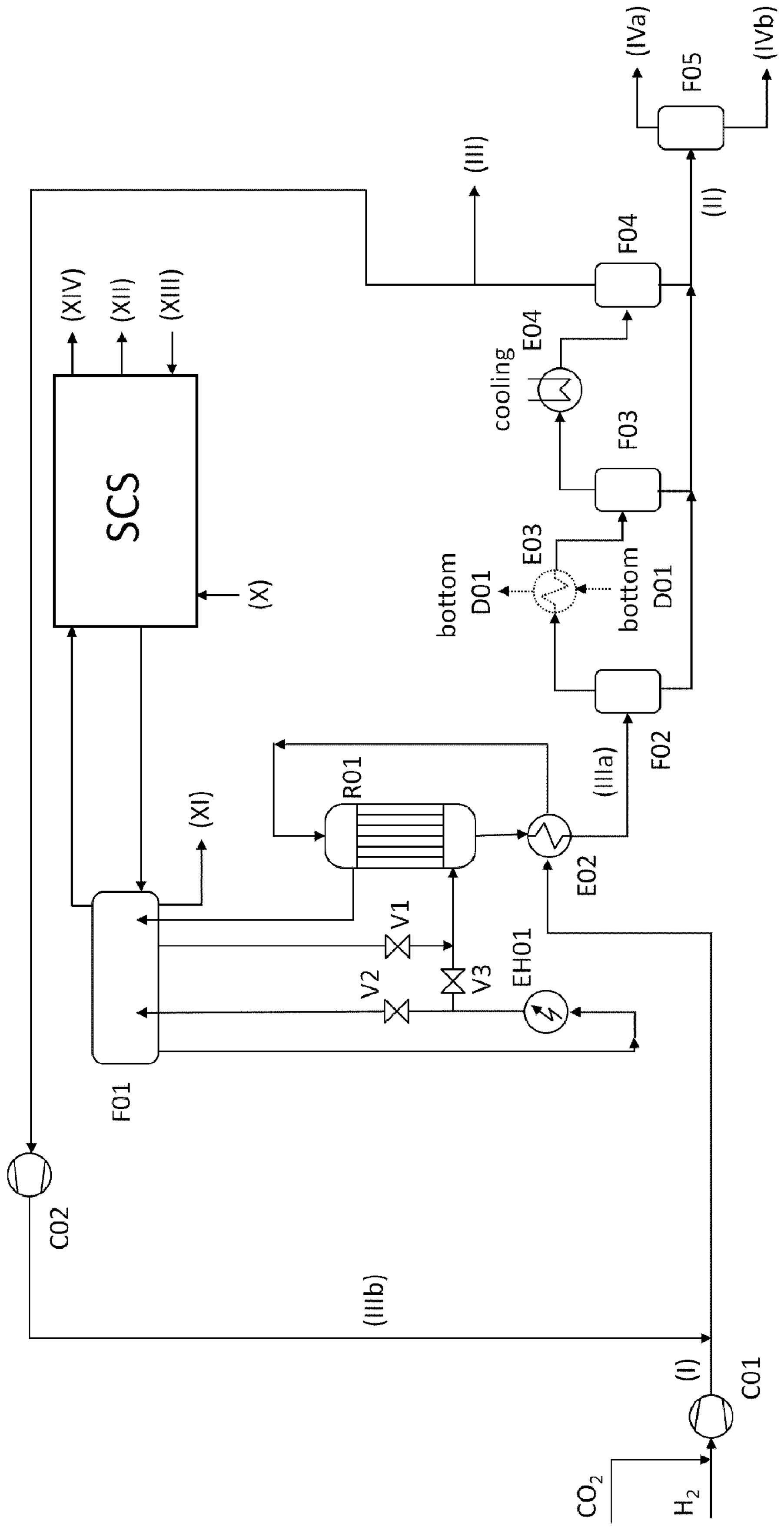

Fig. 5

Process flow diagram, part 2 of 2 (inventive)

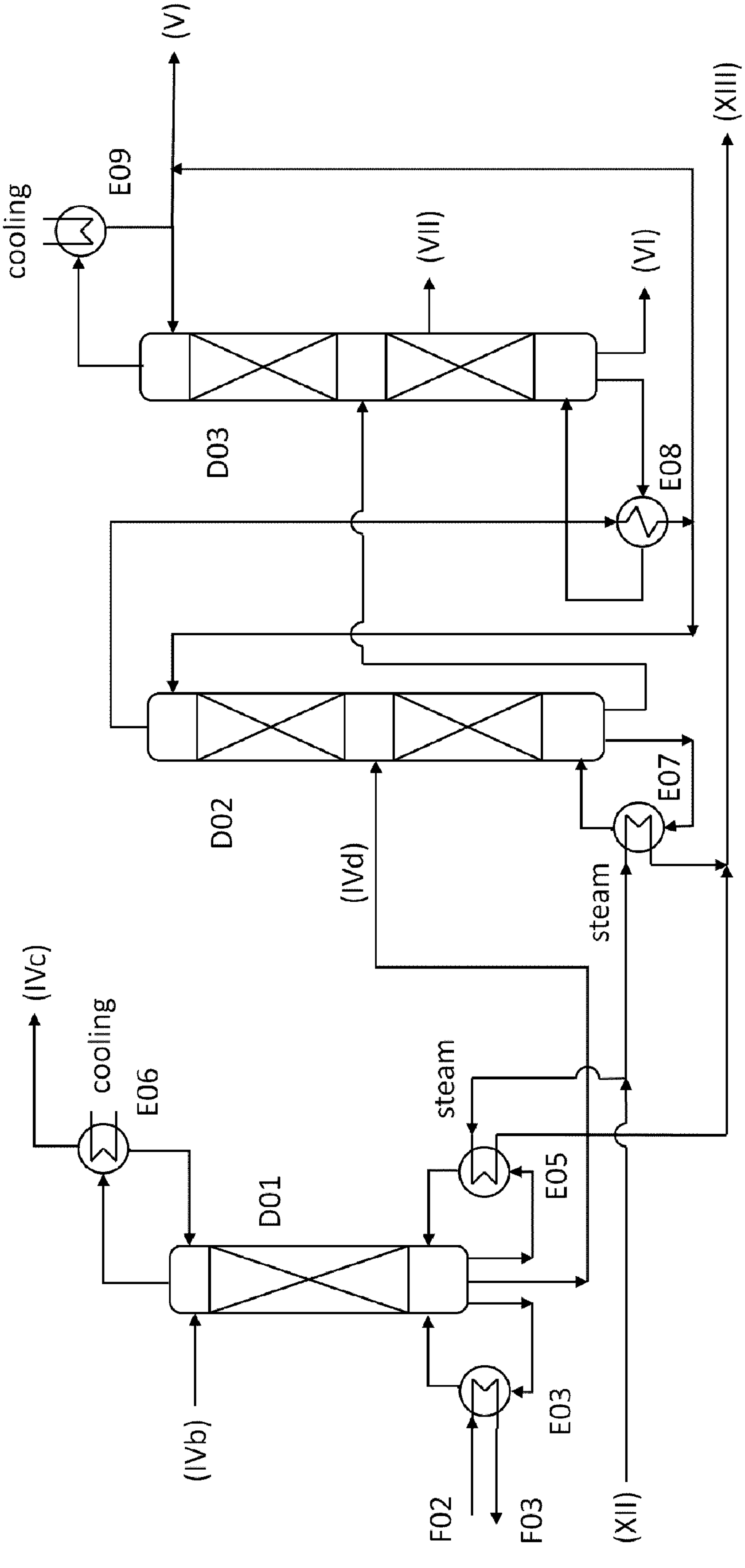

Fig. 6

Process flow diagram, part 1 of 2 (comparative)

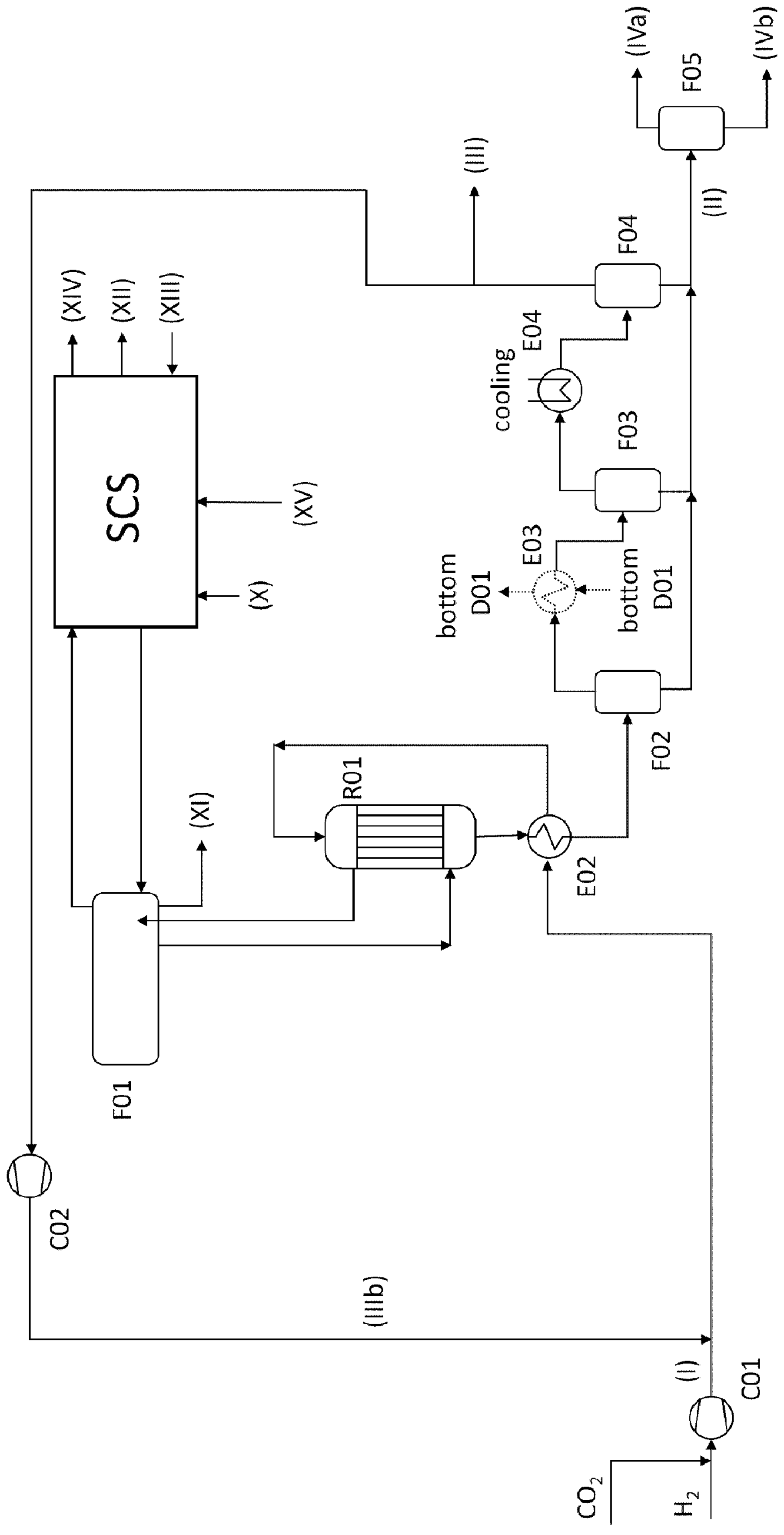

Fig. 7

Process flow diagram, part 2 of 2 (comparative)

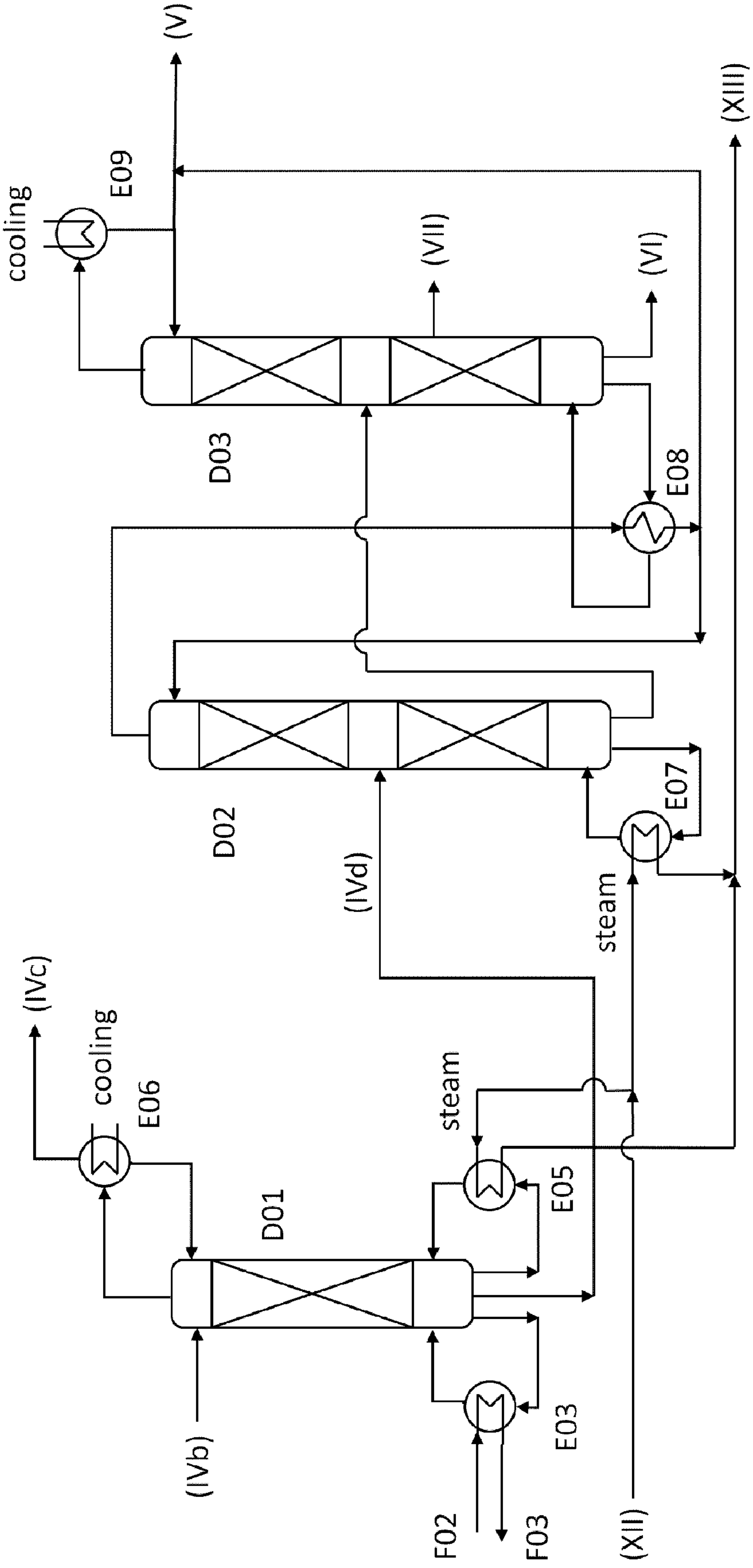

Europäisches Patentamt
European Patent Office
Office européen des brevets

# EUROPEAN SEARCH REPORT

Application Number
EP 21 21 4263

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br><br><br>Y | KOYTSOUMPA EFTHYMIA IOANNA ET AL: "Flexible operation of thermal plants with integrated energy storage technologies", HEAT AND MASS TRANSFER, SPRINGER BERLIN HEIDELBERG, BERLIN/HEIDELBERG, vol. 54, no. 8, 31 August 2017 (2017-08-31), pages 2453-2460, XP036561311, ISSN: 0947-7411, DOI: 10.1007/S00231-017-2148-7 [retrieved on 2017-08-31]<br>* figure 4 *<br>* page 2457, paragraph 2.3 - page 2459, paragraph 3 *<br>----- | 1,2,4-7, 10-15<br><br><br><br><br><br><br><br><br><br><br>3 | INV.<br>C07C29/151<br>C07C31/04<br>B01J19/00 |
| X | WO 2020/150247 A1 (SABIC GLOBAL TECHNOLOGIES BV [NL]; SCHROER JOSEPH WILLIAM [US] ET AL.) 23 July 2020 (2020-07-23)<br>* paragraph [0101] *<br>* paragraph [0152] *<br>* paragraph [0163] *<br>* figures *<br>----- | 1-15 | TECHNICAL FIELDS SEARCHED (IPC)<br>C07C<br>B01J |
| X<br><br><br><br><br><br><br><br><br>Y | MIGNARD ET AL: "Processes for the Synthesis of Liquid Fuels from CO"2 and Marine Energy", CHEMICAL ENGINEERING RESEARCH AND DESIGN, ELSEVIER, AMSTERDAM, NL, vol. 84, no. 9, 1 September 2006 (2006-09-01), pages 828-836, XP022536202, ISSN: 0263-8762, DOI: 10.1205/CHERD.05204<br>* page 829, column 2, paragraph 3 - page 830, column 1, paragraph 2; figure 1 *<br>* page 834, column 1, paragraph 1 - paragraph 5; table 2 *<br>----- | 1,2,4-15<br><br><br><br><br><br><br><br><br>3 | |

-/--

The present search report has been drawn up for all claims

2

| Place of search | Date of completion of the search | Examiner |
|---|---|---|
| The Hague | 23 May 2022 | Patteux, Claudine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 21 4263

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 10 040 738 B2 (CASALE SA [CH]) 7 August 2018 (2018-08-07) * figure 1 * * column 2, line 49 - column 4, line 45 * * examples * ----- | 3 | |
| A | EP 3 730 473 A1 (SABIC GLOBAL TECHNOLOGIES BV [NL]) 28 October 2020 (2020-10-28) * paragraph [0017] - paragraph [0019] * * paragraph [0028] * * paragraph [0099] * * examples * ----- | 1-15 | |
| A | CN 103 890 236 B (BUSSE KARL-HERMANN) 14 September 2016 (2016-09-14) * figure 2 * * paragraph [0089] - paragraph [0143] * ----- | 1-15 | |

TECHNICAL FIELDS SEARCHED (IPC)

The present search report has been drawn up for all claims

2

| Place of search | Date of completion of the search | Examiner |
|---|---|---|
| The Hague | 23 May 2022 | Patteux, Claudine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 21 21 4263

This annex lists the patent family members relating to the patent documents cited in the above-mentioned European search report. The members are as contained in the European Patent Office EDP file on
The European Patent Office is in no way liable for these particulars which are merely given for the purpose of information.

23-05-2022

| Patent document cited in search report | Publication date | Patent family member(s) | Publication date |
|---|---|---|---|
| WO 2020150247 A1 | 23-07-2020 | CA 3126607 A1 | 23-07-2020 |
| | | CA 3126676 A1 | 23-07-2020 |
| | | CA 3126697 A1 | 23-07-2020 |
| | | CN 113966380 A | 21-01-2022 |
| | | CN 113993816 A | 28-01-2022 |
| | | CN 114040904 A | 11-02-2022 |
| | | CN 114175450 A | 11-03-2022 |
| | | CN 114466831 A | 10-05-2022 |
| | | EP 3911603 A1 | 24-11-2021 |
| | | EP 3911622 A1 | 24-11-2021 |
| | | EP 3911718 A1 | 24-11-2021 |
| | | EP 3912251 A1 | 24-11-2021 |
| | | EP 3912252 A1 | 24-11-2021 |
| | | JP 2022522968 A | 21-04-2022 |
| | | JP 2022522970 A | 21-04-2022 |
| | | KR 20210117284 A | 28-09-2021 |
| | | KR 20210117295 A | 28-09-2021 |
| | | US 2022119252 A1 | 21-04-2022 |
| | | US 2022119269 A1 | 21-04-2022 |
| | | US 2022119328 A1 | 21-04-2022 |
| | | US 2022126251 A1 | 28-04-2022 |
| | | US 2022127209 A1 | 28-04-2022 |
| | | WO 2020150244 A1 | 23-07-2020 |
| | | WO 2020150245 A1 | 23-07-2020 |
| | | WO 2020150247 A1 | 23-07-2020 |
| | | WO 2020150248 A1 | 23-07-2020 |
| | | WO 2020150249 A1 | 23-07-2020 |
| US 10040738 B2 | 07-08-2018 | AU 2012367104 A1 | 04-09-2014 |
| | | CA 2860895 A1 | 01-08-2013 |
| | | CL 2014001932 A1 | 20-02-2015 |
| | | CN 104105531 A | 15-10-2014 |
| | | EP 2617478 A1 | 24-07-2013 |
| | | EP 2806957 A1 | 03-12-2014 |
| | | MX 354386 B | 02-03-2018 |
| | | MY 167631 A | 21-09-2018 |
| | | RU 2014134399 A | 20-03-2016 |
| | | UA 112341 C2 | 25-08-2016 |
| | | US 2015008116 A1 | 08-01-2015 |
| | | WO 2013110368 A1 | 01-08-2013 |
| EP 3730473 A1 | 28-10-2020 | NONE | |
| CN 103890236 B | 14-09-2016 | CN 103890236 A | 25-06-2014 |
| | | DK 2751307 T3 | 07-12-2015 |
| | | EP 2751307 A1 | 09-07-2014 |

EPO FORM P0459

For more details about this annex : see Official Journal of the European Patent Office, No. 12/82

page 1 of 2

# ANNEX TO THE EUROPEAN SEARCH REPORT ON EUROPEAN PATENT APPLICATION NO. EP 21 21 4263

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2022

| Patent document cited in search report | Publication date | Patent family member(s) | Publication date |
|---|---|---|---|
| | | ES 2553082 T3 | 04-12-2015 |
| | | WO 2013029701 A1 | 07-03-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017103679 A
- WO 2021148262 A


### Non-patent literature cited in the description


- Methanol. Ullmann's Encyclopedia of Industrial Chemistry. Wiley-VCH Verlag GmbH & Co. KGaA, 2012
- Methanisierung. Römpp Lexikon Chemie. Georg Thieme Verlag, 2019
- **VAN-DAL et al.** *Chemical Engineering Transactions,* 2012, vol. 29, 463-468
- **C. CHEN et al.** *Applied Energy,* 2019, vol. 243, 71-90
- Ullmann's Encyclopedia of Industrial Chemistry. Wiley-VCH Verlag GmbH & Co. KGaA, 2012